# EUROPEAN PATENT APPLICATION

(11) **EP 1 736 926 A1**
(43) Date of publication of application: **27.12.2006**
(21) Application number: 06386017.5
(22) Date of filing: 13.06.2006
(51) Int. Cl.: G06Q 50/00, G07F 7/10

(54) **Electronic health book**

(30) Priority: 13.06.2005 GR 2005100293
(71) Applicant: Linax Holdings Ltd.,, Lefkosia (CY)
(72) Inventor: Liamis, Dimosthenis, Athens (GR); Sarris, Nikolaos, Athens (GR)

(57) **Abstract**

The book includes the holder's identity data, personal medical information (blood group, allergies, etc) insurance data (coverage, verifications, etc) and the full medical history (examinations - prescriptions - referential - laboratory results - medical leave - disabilities - operations and results, appropriately classified) with capability of storing text, images and electronic data from medical equipment.
It gives the capability for a specific category of users (e.g. Regional Hospital Doctors) to transfer the patient's medical information, through the Internet to central Hospitals, or University centers supporting them through Remote Medicine. In this manner emergency and difficult incidents are handled in remote areas with success.
The storage means of the electronic health book may take the form of a USB flash memory stick, a re-writable CD or DVD, or any electronic means of small dimensions, possessing large memory capacity.

## Description

This invention refers to the creation of an electronic health book in the form of an electronic card of sufficient capacity, or in the form of a form flash-drive (usb.).

To this purpose a complete integrated operating system is developed that covers the entire spectrum of Public and Private Health. The objectives of the system are:
1. Improving of the Medical care provided for the citizen and its guarantee against medical errors which, very often, turn to be fatal for the patient and which, as a rule, are due to deficient information provision to the doctors regarding the full and updated "until yesterday" medical status of the patient.
2. Providing the Doctor (in a wide sense: doctor-hospital diagnostic center) with the tool that will help him carrying out a correct and fast diagnosis, in order to avoid medical errors that cost lives but also to provide the right treatment, or intervention.
3. Reforming of Public insurance institutions that will be exempted from false and inaccurate expenses due to medical action and pharmaceutical expenses because with the application of this system there will be immediately control and cross-checking of all expenses automatically. This is achieved by the system because in order for the Doctor - pharmacist - hospital - diagnostic center to be paid, from the any insurance fund should dispatch Electronically via the internet, the charge for the services it provided to the insured, together with their justification, and their cost, simply and easily by pressing a key. This information is automatically crosschecked at the terminal of the insurance institution, per doctor - insured - drug store - hospital - diagnostic center and the expenses are cleared automatically with the assistance of special software. This system abolishes the endless bureaucracy, which on the one hand is crosschecked with difficulty and on the other requires thousands of man-hours and personnel with doubtful results. Moreover the doctors - pharmacists and nursing institutions will be paid in time and validly without discomfort and delays as it happens today. The profit of the insurance funds from the correct settlements, the repeated examinations, which now will disappear, the savings in personnel, it is calculated that will cover the greater part of their deficits today with a two-sided profit for the fund, the insured, and the medical and paramedic health circuit.
4. The insured should be able to meet his obligations to the insurance fund immediately and easily without having either him or his relatives (when he himself is hospitalized and he cannot be moved) to produce the required certifications from doctors - diagnostics - drug store receipts etc in order to pay for his hospitalization to the fund he belongs to. All these processes are carried out by the system and are performed immediately at the moment the medical action is made through an electronic dispatch via the Internet, of all the required documents - certifications - receipts by the one who carries out the medical action. Moreover the validity update for the book can be made automatically by the system as soon as the insured produces it to any doctor - pharmacist - hospital.

Briefly the electronic health book will include the following
✔ The demographic data of the holder
✔ Form E111 (or similar), necessary for the medical coverage of the citizens of the E. U.
✔ The entire medical history of the holder
✔ The holder's medical insurance information.
✔ All medical opinions
✔ The pharmaceutical treatment updated with regard to prescriptions but also the purchase of medicines from the particular drugstore they were bought.
✔ The illnesses suffered during the life duration of the holder
✔ The radiographs-ultrasound graphs-magnetic graphs and other depiction examinations to which he was submitted from time to time, which he does not have to find them in order to produce them to a doctor at any time. In the case where he cannot find some and have been lost, it will not be necessary to do them again by receiving radiation and by paying himself or his fund unnecessarily.
✔ Exemption of the holder from work duties due to medical reasons (medical leave), which is easy via special addition to be transferred to his department or to his employer through the internet.
✔ Capability is provided for cooperating with special medical packets in order to enrich medical information, as well as for determining medical appointments, etc.

Because of the small size it provides to the user the possibility to have it always with him, just like his credit card his identity card or his keys. Thus in the case of an accident or of an emergency entry in a Hospital, immediately and without losing time information will be provided to the attendant doctor concerning the previous medical condition of the patient, necessary and vital for the correct coverage and medical treatment to be provided to him, e.g. blood group - allergies - heart condition - blood pressure - chronic illnesses etc. All information will be displayed immediately and instantly on the computer screen of the Hospital, and moreover on that of attending doctor who will examine the patient and who have obtained the special program for doctors EHB - doctor.

The data will be entered initially into the EHB by special medical centers contracted with the system and it will be enriched during every visit the patient will make to his doctor. It will also be possible to enter into the book important radiographs as well e.g. heart triplex and bone fractures or deformations which will be recalled instantly so that correct and immediate treatment is given not only in the Hospitals but also by a private doctor, since he will have a full history record of the patient. Thusly medical errors due to lack of information provided to the attending doctor will be completely abolished.

Through the assistance of special software capability is provided for reading and recording data in the electronic book for updating the medical history record (addition of a new examination of a medical action), or for changing the holder's medical data (Marking an important illness or allergy ascertained). Also, via the internet, a capability exists for verifying the book by the holder, and dispatching of the examination financial data a doctor performed, so that his monetary compensation is made possible without having to visit the offices of the insurance institution.

Capability is also given to the attending doctor, through a special addition, to support the **user doctor's** communication with the centers that support him and cover him via remote medicine during the execution of his duties, (rural infirmaries, etc).

The tracking down of the EHB in cases of emergencies, when the holder is unconscious, by the stretcher bearers, or by the hospital to which he is taken, is possible through the tracking down system, which is optionally added to the EHB. The receiver of the sound system will be placed in the case of the EHB, it will be activated by a transmitter the stretcher bearers and the hospital possess and it will be durable and waterproof. In the case where the holder wishes it, he may obtain himself as well the corresponding tracking down transmitter, so he may find the EHB easily when he has lost it, or he does not remember where he placed it. This is especially useful for persons with special needs e.g. blind persons, overaged, etc.

Until to today these processes are realized by the use of insurance books in printed form, which between others have also the following two disadvantages:
a. they cannot store a large volume of information for the medical history record of the holder, the detailed opinions of the various doctors or of the diagnostics and of the nursing institutions, treatments, the medicines given to him, the interventions he has been submitted to and informative depicting material, such as radiographs, etc.
b. they compel insured persons and doctors to visit the offices of the insurance institution in order to carry out many work tasks, such as the verification of the book, the verification of examinations and monetary compensation.

Also the electronic systems that until today are used in different European countries and which many of them have been patented, use storage means the simple or smart electronic card which though have the following disadvantages :
**a.** the data that can be stored on the card is very few and refers mainly to the holder's code number, his demographic data, and few health characteristics. Thus no capability is given to the doctor to have the full medical image of the patient **immediately and easily**, concurrently they provide the capability for drawing out more information through the Internet, from a central database where medical records for all insured are kept more fully. The existence though of a central database, in which medical data are maintained, comes in direct contrast with the Principle of Protecting Personal Data
**b.** the doctor's briefing is not carried out immediately and fully since in order to draw out the medical information he needs for every patient he must be connected through internet with the central database where the medical data are kept. This process is time consuming, since for receiving huge files a corresponding amount of time is required. It has a high cost because the doctor the pharmacist, and generally the user must on a permanent basis be connected to the internet, it requires a high cost for installing and maintaining electronic configurations and hardware at the center for maintaining the files of all the citizens. It is also unreliable because at the moment the doctor needs the network might be out of order or not available, with serious consequences especially during emergency incidents.

The advantages of this invention against the remaining similar electronic systems are:
1... that **it does not use a Central database** and consequently it does not clash with the **Personal Data Protection Principles.**
2... its holder has in his absolute possession all his personal Medical data and he can and only he can produce them to whoever he wants to.
**3...** All holder's medical information is contained in a book of small dimensions (size of a small key), including the radiographs, ultrasounds, axial graphs etc. which constitute necessary information for a doctor in an emergency incident but also to primary medical care so that accurate medical opinion is made easy and the provision of pharmaceutical treatment is made safe, which will be compatible with possible sensitivities of the holder to pharmaceutical substances that can be turned out to be fatal on a daily basis.
**4...** All medical visits, opinions, illnesses, interventions he is subjected to, pharmaceutical treatment, vaccinations etc are recorded, so that it is not necessary for the EHB holder to remember, being or not unconscious, the details and to pass them over correctly to the doctor in order to have the right care.
**6...** The drug prescription is executed electronically by the pharmacist in a reliable and valid manner regarding pharmaceutical preparations, and the charging for the drug to the insurance institution is made automatically as well as the participation of the holder to the cost, rendering the pharmacist's job simple and reliable.
**the 7...** The charging data for medical visits, hospitalizations, but also for drug sales are transferred immediately and with accuracy to the insurance institution through the internet, in a very short time, solving thusly the economic problem of the insurance funds, which is due to inaccurate, excessive charges, since through a special program the charges of doctors - diagnostic centers - hospitals - drug stores - holder are fully cross-checked and controlled automatically fully by the system. By this method the insurance funds will greatly save personnel which waste infinite man-hours in order to control and cross-check medical and pharmaceutical expenses today without substantial result.
**8...** And naturally no connection with the internet is required so that the doctor is updated for the patient's medical data, a fact that has all the previously mentioned disadvantages.
**9...** The updating of the insurance institution in the case where the network is not available is performed by the system of line handling thusly the case of circumstantial weakness of the internet, concerning always the dispatch of financial data, which do not bring about a consequence to the immediacy of the patient's medical coverage, since all medical data are stored in the EHB of the patients and not in a central database.

The storage means for the electronic health book may take the following forms:
1. electronic data storage card connected to a USB port of a common Personal Computer (USB flash memory stick).
2. Credit card re-writable 8 cm diameter CD or DVD.
3. any electronic means of small dimensions, possessing large memory capacity

The necessary hardware for providing the integrated "Electronic Health Book" service is the following:
1. Personal computer, with a CD/DVD recording device in the case where the second storage means option is used and an usb port, elements all modern computers have.
2. Special software that will fulfil the processes of reading recording the card and of commun9ication via the internet with the insurance institution.

The electronic configuration of the card-book and the first recording of the insured personal data are carried out by special software that ensures the safe codification of the data so that possible creation of bogus books is avoided. The software for reading the books has the capability of recognizing its authenticity, by the use of an appropriate codification of the book holder's identity. The book will contain data for the holder's identity, personal medical information (blood group, allergies, etc), insurance information (coverage, attestations, etc) and the full medical history record with a capability for storing text, images and electronic data from medical equipment, the pharmaceutical treatment, the medical actions carried out though out his life, the vaccinations, the sick leave granted to him etc appropriately classified in corresponding electronic pages.

Every insured person will be able to carry out any transaction with the insurance institution (such as revision of a book, examinations and financial compensation) through the internet, as long as he obtains the previously mentioned electronic configuration (a computer with access to the internet and software provided on a cd as well as all the corresponding computer programs). If he does not want to transact through the internet he will be able to bring his electronic book forth to the insurance institution and to carry out there any transaction electronically quickly and reliably.

For every medical examination at a private doctor or hospital under contract with the insurance institution, the insured person will produce his electronic book which they could read and update there with the medical examinations performed, through the electronic reading/recording configuration. Also, the doctor or the hospital will enter in the book the examination or the hospitalization and he will be informed immediately for the corresponding insurance coverage of the holder, so that the charging for the necessary amount is carried out. For the compensation of the doctor / hospital he will be able to address the insurance institution either through the internet, or at its offices, by providing a codified electronic file which will be created during the transaction with the insured person and will contain all necessary information for completing the compensation.

### TECHNICAL DESCRIPTION

More specifically, the EHB will have the following technical characteristics:
- Its architecture is structured in a multilevel stratification of components (n-Tier architecture), which simulate the logical and physical entities by ensuring a reliable and secure operation in all levels.
- The independence of its interactive components guarantees the openness of the system not only regarding its interconnectivity with other information systems, but also its ability to operate over different operating systems.
- Intra-system operation is ensured through advanced and generalized APls, able to interact with legacy data, with large scale systems as well as with infrastructures of the existing network, ensuring compatibility with any Relational Database.
- The design of the architecture follows the principle of component reusability, not only for operations on the application level, but also on the user level.
- Its architecture makes use of multi-threaded programming processes by offering increased performance and maximum utilization of system resources.
- The composition of all of the above implements a very wide service network, at the disposal of the final user, accessible with the minimum possible operating requirements from him (on the hardware and software level)

The infrastructure of the EHB has been constructed by the use of the most advanced state-of-the-art technology the software industry and the academic community adopt today (.net Framework, XML, HTML, Web Services), showing particular sensitivity in technological and medical standards by ensuring in this way the defacto conditions of intra-system operation, portability and multi-language use. The technologies operate in such a manner so that the EHB is able to be expanded to multiple audience business rules and languages, and to respond to the creation of integrated services during the complex allocation of 'cognizant objects' to entities such as [Customer-Person Insured], [Associate-Doctor], [Employee-Worker] which comprise the Medical Organization.

Specifically the EHB innately supports the following standards:
Network Protocol: TCP/lP, SSL, HTTP, HTTPS, WEB SERVICES,
Technologies: ADO .[NET] FRAMEWORK, HTML, DHTML, XML
Database connectivity: ADO .NET FRAMEWORK OLE DB FOR SQL/ORACLE OR ODBC

The back-end of the EHB regarding Data Storage is serviced by a Relational Database Management System (RDBMS).

The development of the EHB functionality is based on a fully unified organization platform for recording medical services, distributed on many levels (Data tier - Business Logic Tier - Presentation Tier) and more specifically:

### Data Tier- Relational Database

The EHB data are stored in three different ways:
- Inside the EHB they are stored in XML form (encrypted)
- The data for financial compensation of doctor / pharmacist / diagnostic center are stored in its computer in a database - specifically in the light and free version of MS SQL SERVER - MSDE
- The application data of the insurance institution are stored in a central computer in a database - specifically MS SQL SERVER or ORACLE

In the all objects that the EHB uses corresponds an "Insert" - "Update" - "Delete" (Import-briefing-deletion) process. No datum is ever written immediately in any database table.

This level communicates exclusively with the Business Logic Tier, using a common interface (Data Brokerage Engine) that undertakes the regulation of requests made by different subsystems and of the load reduction on the client computer and on the network.

With regard to the interconnection and communication of doctors / pharmacists / diagnostic centers with the application of the Insurance Institution for the transfer of the necessary medical / financial data this is performed via the WEB SERVERS (internet services).

### Business Logic Tier

Business Logic Tier is responsible not only for carrying out the requests from the Presentation Layer but also for accessing the EHB data. Its interface with the Presentation Tier guides the requests made by the users through the interface environment to the relevant operating units, by returning the relevant results to the presentation layer.

### Presentation Tier

The Presentation Tier is the interface of the user with the EHB. It communicates exclusively with the Business Tier for any operation on the Data Tier.

### Description of security mechanisms

During the design of the EHB special emphasis has been given to the issue of data security due to the sensitive personal information involved.

Matters of safety in the specific system are introduced and handled on the following levels:
- Internet security for the application and data of the insurance institution: The application of the Insurance Institution has been design in such a manner as to operate without any problems behind the Internet protection for application isolation (Firewall) and data communication with Web Services ensures the integrity of data received. The hardware to be used for the Internet protection of the insurance institution will be decided in agreement with the Assigning Authority depending on the present status and the requirements for its use. In any case if no sufficient protection exists already modern hardware of firewall technology will be installed (such as Cisco PIX 515E) and a modern monitoring system for internet transaction aiming to track down malign actions (intrusion Detection Systems) (such as Cisco 4210).
- Protection of personal medical data inside the EHB: The EHB applies the RC2-128bit technology of encryption which ensures the protection of sensitive personal data of the user and in the same way it excludes also the creation of bogus EHB.
- Protection of economic compensation data maintained by the doctor / pharmacist / diagnostic center applications: The application users are predetermined and are certified by entering the unique code given to them during system installation. The data is stored encrypted while its integrity is unbreakably ensured by the Insurance institution through the cross-checking of the corresponding data stored in the EHB of every person insured. This comparison is performed every time the person insured renews its coverage through EHB revision, where the medical action data in his book are cross-checked with the corresponding ones dispatched to the institution by the doctors, pharmacists and diagnostic centers that have provided treatment to the person insured. **With this method the prevention of illegal prescribing is guaranteed since any effort will be tracked down automatically through the first (automatic) cross-check of the prescription data and the book.**

Consequently, the EHB ensures:
- the authentication: That is to say the control of identity authenticity for the data exchange parts through user roles and access control.
- the authorization: That is to say the guarantee of authorized user access in all applications, as well as the EHB provides an integrated security system and user access by providing the capability of determining users, groups, roles and access rights, adapted immediately to the organization and operation structure of the insurance institution.
- the integrity: since it is guaranteed that the data are not subjected to alteration due to the encryption and cross-checking performed between the data received by the insured persons and the cooperating institutions.
- the non-repudiation: The user should not be able to deny his attendance in data exchange (e.g. recording transactions in the database) as the transactions are recorded in the base of the insurance institution, as well as in the vases of the cooperating institutions.
- the audit: Since every access, modification or processing of data in the EHB can be audited, by whom it was made and when (with the use of timestamps.

### Functional Description of the Proposed System

For the general comprehension of the proposed system the following brief description which summarizes the general characteristics and advantages of the proposed **EHB** is introduced.

**What is it:** The new storage method for the entire medical history record of the holder, at the size of a key, with integrated management capabilities for all institutions involved (insurance institution, doctor, pharmacist, hospital, etc). The storage means is electronic memory connected to a USB (USB flash memory) port.

**How does it function:** With its connection to any personal computer the holder's data is displayed as well as his entire history record, i.e.: illnesses, examinations, results and insurance coverage data, with capability of storing text, and image by authorized users (eg doctors). Selected data may be protected by a personal code number.

**Benefits of Holder:** The holder has all his medical data concentrated. There are concentrated all his illnesses, drugs, and allergies so that a case of Medical error is nullified, and the attending doctor is updated correctly, in time and validly for the holder's full Medical History Record.

**Benefits of Doctor:** The personal doctor and the hospital have at their disposal the full medical history record of the patient, with immediate indication of critical information for cases of emergency incidents

**Benefits of doctor, Hospital, Pharmacist:** The doctor, the hospital and the pharmacist can be compensated electronically, via the Internet, without needing to visit, neither them nor the patient, the insurance institution.

**Benefits of Insurance Institution:** By the use of a key the financial data of the Medical actions are automatically transferred to the insurance institution, which now has the data of its interest registered, and it manages it with ease. The data may be displayed per holder, doctor, pharmacist or hospital. With this method apart from the full immediate and accurate updating of the organization regarding the expenses ensuing from the entire health system it covers, a capability for full audit of the expenses, as well as the electronic settlement of the amounts owed is given with the minimum preoccupation of the Organization's personnel. **Therefore very significant benefit will ensue as a function of a multiple activity audit.**

**What can be included in the book:** The form E111 required by the E.U., as well as other data required by the institution use regarding Persons with special needs, disabilities examinations and committee opinions (with special reference to independent pages) disability status changes etc.

The structure of the operating system allows the addition of additional requirements of the institution participating in the application program.

A detailed description for the EHB operation follows by analyzing the necessary installations during the preparation phase, as well as all the possible actions during full system operation.

### Preparation Phase

Before starting the operation of the integrated system the following actions must be necessarily made:

### Installation of insurance institution application.

The application for receiving all the necessary data for medical actions must be installed in the Insurance institution depending on the configuration it wishes to have.

This application receives the data sent by doctors, pharmacists and diagnostic centers through the internet and enters them in a database of the department, which may be either SQL SERVER or ORACLE. In the case where it is not installed it must be purchased and installed, while in the case where there is one installed simply it must be connected with our application which will be adapted to the existing base structure. It is noted that the data hosted by the base will be necessary for the financial management of medical actions of the insured. **It is not necessary for sensitive medical data of the insured to be maintained in the base since these will be stored in the Electronic Book every insured person will have in his possession, so that the integrated system must respect the personal data of the insured and not to clash with the national and community legislation regarding personal data.**

### Creation of an Electronic Book for the Insured

The application of the integrated system requires for a start the procurement of a storage means, which will host medical data of the insured, hereupon an electronic memory connected to a USB (USB flash memory) port.

The electronic arrangement for every book follows as well as the entry of demographic and insurance data of the insured in encrypted form. This process will be carried out by the company constructing the EHB based on data provided by the insurance institution.

Next the book is delivered / dispatched to the holder together with his personal access code number, ready for use.

Together with the EHB a c.d. is delivered to the holder as well which installs, via an easy and completely simple method, in this personal computer, the special program for holders which allows **only** the reading of EHB contents. The pin is delivered through which entry in the system is allowed.

### Installation of application in collaborating Doctors, Pharmacists and Diagnostic Centers

Every doctor, pharmacist and every diagnostic center will have its own copy of the application (with its own access code incorporated) which will provide the capability of reviewing the contents of the electronic book of every insured person, of entering new data and of dispatching data to the insurance institution, depending on the usage rights of every one, as described in detail in this section below.

This application, while it will be able to be installed by him himself, it would be preferable to be installed by specialized personnel which will provide as well brief training for the use of the application.

### Operation Phase

Having delivered the Electronic Health Books to the insured and having installed the necessary applications, as described in the previous section, the Electronic Health Book is ready for operation. The insured person who wishes in addition to enter his previous medical history record inside the EHB can do it by producing to his personal doctor the corresponding medical proving documents or by carrying out new examinations which prove the illnesses he wishes to record. The doctor, when he is sure that the medical data presented to him are true can undertake the responsibility to enter into the EHB of the insured. **It is noted that the application always records for any EHB data change, who was the doctor that entered it.**

Next, new data are entered in the EHB for every new medical (and pharmaceutical) action, continuously composing in this manner the medical history record of the insured, by registering in general lines the following:
- Visit of an insured person to a doctor of any specialty, recording of the diagnosis and dispatching of financial and other data required to the insurance institution for the financial settlement of the doctor.
- Referrals to a doctor of another specialty for specialized examinations and dispatching of all the required financial data to the insurance institution.
- Prescribing of drugs and therapeutic treatment.
- Approving a prescription required with additional documents.
- Implementing a prescription (purchase of drugs from the drug store) and dispatching of data regarding the drugs sold by the pharmacist to the insurance institution.

The detailed operation of the above actions is described in every detail in the remaining part of this section, with the help of attached application images:
1. **Image 1** verification of doctor's identity by the system via the code number needed so that reading of the EHB is allowed to him.
2. **Image 2** demographic and insurance data of the EHB holder
3. **Image 3** key by the selection of which critical data for the health of the holder are displayed
4. **Image 4** the critical health data as required by the doctors for the coverage of an urgent incident.
5. **Image 5** consolidated list of all medical examinations to which the EHB holder is subjected to throughout his life
6. **Image 6** the doctor, by selecting from the previous one, one of the examinations, the examination details are displayed which the specific doctor that performed it wrote.
7. **Image 7** The selection of this key provides the capability to the doctor to record all the data of the new examination
8. **Image 8** a list appears with all the data required to be recorded by the doctor so that the description of the medical examination is considered complete. Most data are pre-installed in the system so that simply are selected by the doctor from lists and are listed automatically
9. **Image 9** data entry of laboratory measurements (a full pre-installed list is provided with the min. max normal values for all measurements)
10. **Image 10** Entry of depicting examination (radiograph-ultrasound etc.) by the doctor
11. **Image 11** Drug prescription by the doctor
12. **Image 12** referral for further examinations by the doctor
13. **Image 13** registration for granting medical leave by the doctor
14. **Image 14** option for dispatching the already registered financial data of the examination to the insurance institution of the holder
15. **Image 15** confirmation for the dispatch the doctor's data to the insurance institution
16. **Image 16** purchase of drugs from a drugstore, a consolidated list of prescriptions by different doctors with an indication for pending prescriptions.
17. **Image 17** detailed presentation of the selected prescription with quantities and detailed data for the pharmaceutical preparation.
18. **Image 18** drug data the doctor wrote and taking instructions
19. **Image 19** consolidated the full medical history record of the holder per category with selection capability.
20. **Image 20** selection from the history record: full data of medical leave
21. **Image 21** ALLERGY selection consolidated list of the time to time ascertained allergies by the doctors.
22. **Image 22** with the selection of a specific allergy full doctor's data are displayed.
23. **Image of 23** consolidated disability table with the selection of a specific recording the details are displayed automatically.
24. **Image 24** consolidated accident-injury table, throughout the holder's life, always a capability is provided for selecting a specific incident and for automatically displaying all recorded medical details
25. **Image 25** accident details selected from the previous table.
26. **Image 26** on this page capability is provided to the holder to change some of the demographic data e.g. the house address through the last doctor (only a doctor has the right of recording in the EHB) and to automatically update the insurance institution as well, without the holder himself needing to wait in queues and to waste time.
27. **Image 27** a page containing in consolidation all vaccinations the EHB holder has performed throughout his life
28. **Image 28** on this page vaccination detailed information is displayed selected from the previous page.
29. **Image 29** this image presents in consolidation the values of VITAL POINTS of the holder which from time to time have been measured by various doctors and automatically by the system have been placed onto this page.
30. **Image 30** on this page a list with the names and specialties and the dates when the EHB holder has visited doctors from time to time
31. **Image 31** by selecting in the previous table of a specific doctor his full data are displayed so that communication with him is made possible
32. **Image 32** On this page every illness per holder system is succinctly displayed, so that a doctor may see at a glance the ascertained illnesses in the system he wishes, to see in order of precedence e.g. the heart, blood circulation system, etc.
33. **Image 33** a page for pregnancies a woman had throughout her life in a consolidated table.
34. **Image 34** all microbiological examinations are automatically displayed with their prices and registration date.
35. **Image 35** hospitalizations in hospitals with dates and details, following a selection as in the following page
36. **Image 36** all details of selected hospitalization or intervention.
37. **Image 37** all children's illnesses that the holder sustained recorded by doctors.
38. **Image 38** following a selection on the previous page the details of a specific children's illness and its medical characteristics are displayed.
39. **Image 39** the family history record of the holder full and valid, written by his family doctors.
40. **Image 40** finally the death certificate in the form required by the Greek Law, which as soon as it is compiled is dispatched automatically to the insurance institution and again automatically the Electronic book is cancelled so that it cannot be used further
41. **Image 41** death certificate necessary information required by the Greek Law
42. **Image 42** similarly as above
43. **Image 43** similarly as above
44. **Image 44** all the insurance data of the holder and their changes.
45. **Image 45** a page that includes the pregnancy table for women.
46. **Image 46** a page that has a consolidated table of surgical interventions the EHB holder was subjected to
47. **Image 47** following a selection in the previous table detailed information is displayed regarding the specific intervention the doctor selected
48. **Image 48** this page is displayed when the doctor belongs to the human resource of some hospital, as to the rest the same pages appear in the same order, which were described for the private doctor but with the addition of the hospital data on every page.
49. **Image 49** on this page the prescriptions given from time to time to the holder with quantities, dates and the doctor data in full are displayed. Also the execution or not of the prescription is listed automatically.
50. **Image 50** the pharmacist by selecting a specific prescription from the previous table, its data are displayed in detail, and the cost of every drug, as well as the participation of the insured to it. The pharmacist by selecting the drug the doctor wrote automatically executes the prescription of every preparation. By returning to the initial menu and by pressing the key I.I. he dispatches the prescription data execution, to the insurance institution.
51. **Image 51** from this point the installed program starts in the insurance institution and its operation is described. Thus when the access code is requested from the employee of the 1.1. in the program, in order for the system to determine the "role" and the duties of the user e.g. financial, managerial, organization manager, etc. depending on his identity access to determined data in the system is allowed.
52. **Image 52** the initial selection menu is displayed providing access to doctor, insured, drug store data etc.
53. **Image 53** by selecting the key general information a general list is displayed of the collaborating with the institution doctors, hospitals, diagnostic centers.
54. **Image 54** by selecting from the list a specific associate his full data is displayed. And we can select (having full access to the system) information or examinations or the prescriptions the specific associate made, and the 1.1. was debited for.
55. **Image 55** by selecting Examinations the full list of the examinations is displayed the specific associate performed (doctor-hospital-diagnostic center, etc) on behalf of the insurance institution, by providing full and detailed information for each examination selected by the user. In the lower part of the page a consolidated table is displayed with the cross-checked (doctor-insured person) charges, amounts owed by the fund to every of its associates, per month-year, where automatically and reliably the settlement of amounts owed by the institution is to its associates is made without any further process.
56. **Image 56** by selecting PRESCRIPTIONS all prescriptions the specific doctor prescribed and who was the insured are displayed. Also information is provided for every prescription i.e. what drugs where written how much they cost, and what is the participation of the insured to the cost.
57. **Image 57** correspondingly by selecting from the initial menu INSURED the user may have a consolidated list of his insured persons with all their information.
58. **Image 58** by selecting specific insured person information of every kind is provided to him depending on the options at the lower part of the page.
59. **Image 59** by selecting INFORMATION all registered data for a specific insured person is given.
60. **Image 60** by selecting EXAMINATIONS all examinations are displayed on a consolidated table, with full data cross-checked with the corresponding entries of the doctors, i.e. examination date - doctor - cost. And the total cost of the insured is displayed automatically (regarding medical fees) per month or year
61. **Image 61** by selecting PRESCRIPTIONS a consolidated table is displayed with all drugs given to the specific insured person and the data of all the doctors who prescribed them and concurrently is checked if they have been bought and what was the cost of every preparation, as well as the participation of the insured to the cost.
62. **Image 62** by selecting RENEWAL LIST a full insurance profile is provided for the insured with the insurance renewals and interruptions, the participation percentages in drugs and examinations etc.
63. **Image 63** by selecting LEAVES a full list of medical leave granted to the specific insured person is displayed and from what doctor or hospital.
64. **Image 64** by selecting leave COMMENTS the justification for granting every medical leave is displayed.
65. **Image 65** by selecting from the initial menu DRUGSTORES a consolidated list of the drugstores collaborating with the institution is displayed.
66. **image 66** by selecting from the previous list a specific drugstore the full data for that drugstore is displayed.
67. **image 67** by selecting PRESCRIPTIONS from the drugstore list we have already selected all prescriptions are displayed that have been executed and concern insured persons of the fund, and to what insured person, with all the details regarding the drug, the quantity, the cost and the participation of the insured person to the cost. Also the monthly or yearly drugstore account is shown and automatically its settlement.

### Visit of the insured person to the doctor

Having placed the **EHB** in full operation every insured person will carry with him the EHB at every medical visit and will deliver it to the doctor so that he enters any data needed in order to describe the results of the visit, as it exactly happens (or should be happening) in the current process by the use of a standard (form) health book. In addition it is noted that it will be recommended to every insured person to carry always with him his personal EHB, so that full knowledge of his history record will provided to medical personnel that may be needed in order to carry out treatment during an urgent incident. This will be now able to be done and practical since due to the small size of the EHB this may be placed on a key holder taking the place and the space of a simple key.

In any case the insured delivers his personal EHB to the doctor, who will enter it into the USB port of his computer, where the application has been installed personalized for the specific doctor. The first window displayed requires the entry of the doctor's personal code number, while his name is displayed automatically (**Image 1),** as well as for reasons of security he is the only one who is allowed to use the application¹
¹ In cases where more than one doctors are housed and use the same computer there is the option of selecting the user via a list selection list.

With the entry of the personal code number the system recognizes as well the user status, which determines the usage rights as they will be analyzed in the remaining part of the section. This status may be one of the following:
1. Insured
2. Doctor²
3. Pharmacist
4. User of Insurance institution
² In the all reports the meaning of a *Doctor* represents not only the doctor in his personal infirmary but also the doctor in the Hospitalization Institution (Public or Private), Diagnostic Center etc. In the second case though, the name of the Institution, center, etc is recorded internally

It is noted that further on the system may (if the Assigning authority chooses to) require the entry of a code number from the insured person as well. In this case there will be a process by which authorized hospital institutions may be able to immediately receive the code number of the insured (through the help desk operating on a 24 hour basis) in the cases of urgent incidents where the insured is unconscious.

### PERSONAL - Demographic Information of the Holder

Having entered all the necessary code numbers in the system the following demographic data of the insured are displayed, in the form shown in **image 2**

The attending doctor already from this first page obtains his selection right as to what exactly wants to see or fill in. There are the following options covering medical actions:
- S.O.S. page
- old examinations
- old Prescriptions
- Medical History Record

### S.O.S. page

This page immediately displays on the Doctor's screen the critical Health data of the holder, which a doctor must know during an urgent incident

By pressing the S.O.S. key **image 3** which is located on the initial page displayed to the user after the introduction of his code number, a brief history record is shown with the necessary data in the case of an emergency incident, **image 4**

### EXAMINATIONS

On this page **image 5**, a brief list with the medical visits and examinations is automatically displayed, which has been submitted by the holder and specifically:
- The date of examination
- The category of examination
- The type of examination

From the list, as shown, capability is given to the user doctor by pressing the key ***EXAMINATION DISPLAY*** to display the details of any examination he will select, so that he can see its data as these have been entered by he himself or by another doctor. Specifically grouped information is displayed under the following categories, as shown in **image 6:**
- Examination data
- Examination measurements
- Depicting examinations
- Prescription list
- Referrals
- authorization

On the same page for the examinations capability is given to the doctor to enter a new examination by pressing one key only **image 7**

In a friendly manner the necessary data are entered such as the category of the examination and its description, which are entered with ease through the categories already entered in the application (parameterization of the system independent to code number).

On the page for entering a new examination, as shown in image 8, there are the following fields:
- Clinical Examination (the findings from the review, hearing, feeling, percussion)
- Opinion (the first impression of the doctor after the clinical examination and the study of the depicting examinations)
- Diagnosis (the illness from which the doctor believes that the patient is suffering from, after the end of the visit)
- Final Diagnosis (the illness **classified according to standard ICD-10** from which undeniably the patient suffers from after the end of the required examinations) and which is also registered in the system.
- Cost of Examination
- Participation percentage of the insured (which may be filled in by the doctor or to be calculated automatically from the pricing data of the insurance institution)

Beyond the above basic data of an examination capability is given to enter also the following information:
- **Examination measurements**: detailed description of the laboratory measurements requested to be carried out by a referral. The list is already entered, from which medical orders are simply selected. As shown **in image 9**
- **Depicting examinations:** similarly the required depicting examinations recommended by the doctor are described in detail, so that final diagnosis and therapy are completed. Image 10
- **Prescription list**: gives the capability of entering the necessary pharmaceutical treatment the doctor selects. It is entered in a manner by which it can be performed with ease by any pharmacist (as the corresponding chapter describes) as shown in **image 11**.
- **Referrals:** Gives to the doctor the capability to define the conduct of the necessary laboratory or depicting examinations, or further the referral of the patient for examination by another medical specialty. **Image 12**
- **Authorization:** the specific option gives to the doctor the capability of filling in the form for convalescent leave, when this is required. **Image 13**

After the completion of filling in all the data the doctor considers as necessary in order to describe the specific visit, by pressing the key ***ENTRY*** the new examination is stored together with all its data in the EHB, while limited data which are necessary for the correct compensation of the doctor are stored also in his own computer codified. Specifically the following are stored:
- The data of the patient he examined
- the type of examination
- the financial data regarding the doctor's compensation by the Insurance institution.

The above data are dispatched to the insurance institution, as soon as the doctor selects to be connected to the Internet and press the corresponding key: **image 14** which is in the initial menu.

With the activation of the dispatch process of the visit data to the insurance institution the system communicates with the pre-selected address of the insurance institution and a confirmation message appears on the doctor's screen: **image 15**

The dispatch of the visit(s) data is completed in a few seconds, by updating the departments of the insurance institution regarding all medical actions not dispatched.

### PRESCRIPTION LIST - Electronic prescription formulation

In the unit PRESCRIPTION LIST the doctor obtains the capability of compiling electronically in an easy and quick manner the required drug order indicated for handling the illness of the insured he diagnosed.

The selection of the appropriate drugs or concoctions is performed easily through a fully pre-installed drug list **(EOF drug list)** which has the ability to be updated regarding the new or cancelled preparations through the internet interconnection with the insurance institution.

The capability is given to the doctor to check the following **image 16**:
- all previous doctor prescriptions, so that he is fully updated on the history record of drug taking
- pending prescriptions
- executed prescriptions

The user doctor has the capability to display a prescription and see in detail the data within by pressing the key DISPLAY as shown in **image 16**
- date of issue of prescription
- name of doctor who compiled the prescription
- name of the auditing doctor
- prescription comment
- name of drug
- quantity
- type of preparation
- quantity that has been bought

By selecting DISPLAY the doctor can see the detailed information for every drug contained in the prescription, as shown in image 17
✔ name of drug (according to the EOF)
   - concoction
   - quantity
   - participation
   - directions
   - stop date for taking the drug
   - remarks

It is noted that in all applications the doctor executes any action, he may read what was written from previous entries of the same or of another doctor without being able to add or remove data or sections.

### HISTORY RECORD

The section HISTORY RECORD from the initial page is composed from the categories below, as shown in **image 18**:
- leave
- allergies
- disabilities
- accidents/injuries
- demographic data
- vaccinations
- persons insured indirectly
- vital points
- doctors
- history record of illnesses per system
- pregnancies
- microbiological examinations
- newborn
- hospitalization in a hospital .
- child period illnesses
- family history record
- death certificate
- insurance information
- childbirth
- surgical interventions

In the **leave** the following are displayed in detail **image 19**:
- leave start date
- leave termination date
- number of days

In the **allergies** the following data are displayed image 20:
- date of entry
- type of allergy
- way of appearance
- name of doctor
- description

By pressing the key DISPLAY the page with all allergy data are displayed, **image 21**

In the **disabilities** the following are shown in detail as in **image 22**:
- date of entry
- description of disability
- percentage %
- comments

In the **accidents/injuries** the following data are displayed as shown in **image 23:**
- date of accident
- description of injury
- name of doctor
- date of entry

By pressing the key DISPLAY the page with all the data regarding accidents/injuries are displayed as shown in **image 24**.

In the **demographic data** the following are displayed in detail as shown in **image 25:**
- father's name
- mother's name
- sex
- profession
- address
- education
- address of residence
- citizenship
- nationality
- family status
- general characteristics

On this page capability is given of changing certain data of the insured e.g. address change, telephone number, etc. and this change is transferred automatically in the first initial page of the holder's demographic data.

On the page of **vaccinations** the following data are displayed, as shown in **image 26:**
- type of vaccine
- date of vaccination
- series number
- name of doctor
- doctor's registration number
- date of entry

By pressing the key DISPLAY the page is displayed with all vaccination data. Image 27

On page **vital points** the following are displayed in detail as shown in **image 28:**
- date and time
- description of measurement
- value
- unit of measurement
- Min
- Max

On the page **Doctors** all the data are displayed of the doctors the holder has visited **image 29** :
- Doctor's surname
- Doctor's name
- Registration number
- Work telephone number
- House telephone number
- Address
- area
- prefecture
- PC

By pressing the DISPLAY the page with the full data of the specific pre-selected doctor are displayed. **image 30**

The **history record of illnesses** shows in consolidation, chronologically and briefly according to ICD-10 all the illnesses of the Holder entered from time to time by the doctors the EHB holder has visited. The entry is made automatically when the doctor fills in the field *FINAL DIAGNOSIS.* The classification into the category of operating system the specific illness belongs to is conducted automatically by the use of a smart logic of entry. Specifically the following are displayed as shown in **image 31**:
- Date of Examination
- Illness
- Category of illness

In the Pregnancies the following data for every pregnancy of the insured are provided **image 32**
- Date of Pregnancy
- Conclusion of pregnancy
- Comments

With the selection **microbiological examinations, image 33** all data of microbiological measurements which have been entered automatically from examinations the holder was subjected to from time to time are displayed in detail and in consolidation, while they can be presented grouped in alphabetic order, per unit of measure, per date, or per any other of the following categories:
- date and time of measurement
- name of measurement
- quantity
- unit of measurement
- lower limit
- upper limit
- category

In the page **hospitalization in a Hospital image 34** the following data are given:
- hospital name
- hospital section
- date of entry
- date of exit
- diagnosis at exit
- cost of hospitalization

By pressing the key *DISPLAY* the page with the full data of hospitalizations in Hospitals are displayed. image 35

In the page **Child Period Illnesses image 36** the following data are displayed:
- date of illness
- description of illness
- comment
- name of doctor
- date of entry

By pressing the key *DISPLAY* the page with full data for illnesses during the child period are displayed, **image 37**

In the page **Family History Record** the following are displayed as shown in **image 38:**
- degree
- history record
- name of doctor
- date of entry
- father
- mother
- grandfather from the mother's / father's side
- grand mother from the mother's / father's side
- brothers/sisters
- other relatives
- spouse

The **death certificate** is dispatched to the Insurance Institution so that the cancellation of the Electronic Book is performed automatically. The death certificate consists of 4 pages which are presented in **images 39, 40,41,41,42,** as it is precisely required by Greek Legislation. Of course it can follow the form of any European or local standard:
On the page insurance data the following information exists as shown in **image 43:**
   - type of insurance
   - start date
   - termination date
   - examination coverage %
   - prescription coverage %
In the page childbirth the following data are shown as per **image 44:**
   - date/time of childbirth
   - duration of pregnancy
   - natural, with intervention, complications
In the surgical interventions the following data are displayed as per **image 45**, 46:
   - date of intervention
   - type of intervention
   - place of intervention
   - name of doctor
   - date of entry
By pressing the key *DISPLAY* the page with full data for hospitalizations in Hospitals are displayed.

### Visit of the insured to a Diagnostic Center

The Diagnostic Center application provides for the dame functionality and structure with that of the doctor with the difference that despite that the application can be used by a doctor during the dispatch of data to the Insurance Institution, the data of the Diagnostic Center will be updated (while the name of the doctor who conducted the medical action will be stored). For this reason the functional description is not repeated which is at all similar to that for the doctor with the difference that the name of the Institution is referred to in all interface images, as shown in **image 47,48**

### Visit of the insured to the pharmacist

The proposed EHB system provides to the Pharmacist the following capabilities / rights, **image 49:**
- Review of the demographic data of the insured
- Brief review of the prescriptions entered in the EHB
- Detailed review of the prescriptions entered in the EHB and are pending

By selecting a pending prescription the pharmacist by pressing the key EXECUTION may enter the drug sold to the insured by simply activating the bar code scanner over the serial number of the drug (**image 50**).

In the same manner the doctor dispatches the financial data for the medical actions, the pharmacist dispatches as well the data of the drugs sold: when he wishes, he is connected to the internet and by pressing the key for the Insurance Institution starts the dispatch of the prescriptions not dispatched during a previous communication.

### Use of Insurance Institution application

With this application, which is installed in the insurance institution, the capability is given to the organization to electronically receive through the Internet information for every Medical Action regarding the entire Health cycle it covers. This information is analyzed per groups of users, Doctors - Hospitals - Diagnostic Centers - Drugstores - Insured Persons and is compiled onto consolidated tables by giving the information cross-checked through formulas so that their distribution and cross-checking is performed automatically.

In order for the authorized user of the organization to be allowed to enter the program, the identification of the unique code number, which is provided by the system and the insurance institution defines, is requested e.g. OPAD, depending on his status. The allowed user statuses are the following image :
- Doctor (controller)
- Administrative executive of the insurance institution
- Financial executive

Depending on the status of the user, access is allowed to all or to limited program fields, which will be determined by the organization during the application study phase **image 51**.

All available fields, in a first approach of their design, are developed below.

When the user selects the menu GENERAL INFORMATION he is asked to determine which of the following fields wishes to activate:
By selecting the field **DOCTORS,** a consolidated list of the collaborating Doctors - Hospitals - Diagnostic Centers - Clinics is displayed with the Code Number and the personal data in brief **image 52**
Via a filter from the consolidated list a specific associate of the organization may be queried **image 53**
By selecting **INFORMATION** for the specific doctor personal or other data that will be requested by the insurance institution to be stored on the specific page

With the selection **EXAMINATIONS** a consolidated list of the examinations is displayed which has been dispatched by the specific doctor to the institution, specifically:
- Category
- date
- the name and surname of the insured.
- the cost of examination.

In the lower part of the table the financial data of the examinations the specific doctor conducted are displayed and specifically:
- The total of the debt which is displayed optionally per month or per year **image 54**

In all fields capability is given of printing every screen

With the selection **PRESCRIPTIONS** a consolidated list with the prescriptions the specific doctor has prescribed is displayed **image 55**.

By selecting a specific prescription the user retrieves the details of the prescription i.e.:
- quantity
- participation
- prescription status i.e. pending or executed

All data are cross-checked with the data the pharmacist has dispatched. With this structure full control and cross-checking between the data for the Medical actions - Prescribing - and drug sales is achieved

At this point the chapter Doctor - Hospital - clinic - Diagnostic Center has been completed where they are handled as identical entities since their activities coincide.

With the selection: **INSURED PERSONS** the user of the insurance institution enters the page for the insured persons of the organization. The insured is handled by the EHB as a separate entity. Capability exists for updating the EHB regarding his insurance status either from his own computer or when he visits a doctor, by connecting through the Internet with the insurance institution he is automatically updates the EHB regarding the insurance status of the holder. **Image 56**

By selecting DISPLAY a list of all persons insured is displayed together with their personal and insurance information. **image 57**

On the menu for the insured persons detailed information is provided by following the following process:
By selecting:
   1. **INFORMATION** all personal information is provided requested by the insurance institution to be on that page. Typically the demographic information of the insured has been entered. **image 58**
   2. **EXAMINATIONS**
      a list of examinations the insured has been subjected to is displayed, where all the necessary details are provided for the examination date - examination category - Doctor's data - financial examination data. Also the total cost for the examinations of the specific insured person is given for the time period requested e.g. per month or per year **image 59**
   3. **PRESCRIPTIONS** the detailed prescription data are displayed by which prescriptions the insured person was supplied following an examination by a doctor in full correspondence with the examination. Also information is given if every prescription is executed or not **image 60**
   4. **RENEWALS** capability is given to the OPAD user to enter - update - or and cancel the EHB of the insured, to enter or to modify the participation percentages of the insured in the medical and pharmaceutical expenses, always by entering the user's signature. When the insured visits the doctor, the doctor is connected through the internet with the institution and automatically updates the book of the insured via an easy to use process. **image 61**
   5. **LEAVE** all data for leave given to the insured by every doctor are entered automatically, i.e. granting date - leave date - doctor information - justification - and comments **image 62.63**.

With the selection **DRUGSTORES** the user of the insurance institution enters the page for the organization's contracted drugstores. As in the other fields capability is given to the user to see the list with the drugstores and through a filter from the consolidated list a query can be made for a specific associate - pharmacist of the organization. **Image 64**

The user may see the general information regarding drugstores by pressing the corresponding key. **image 65**

Finally the user can see also the prescriptions in a detailed list the specific drugstore has executed as well as the financial data the organization is interested in per month / year. **Image 66**

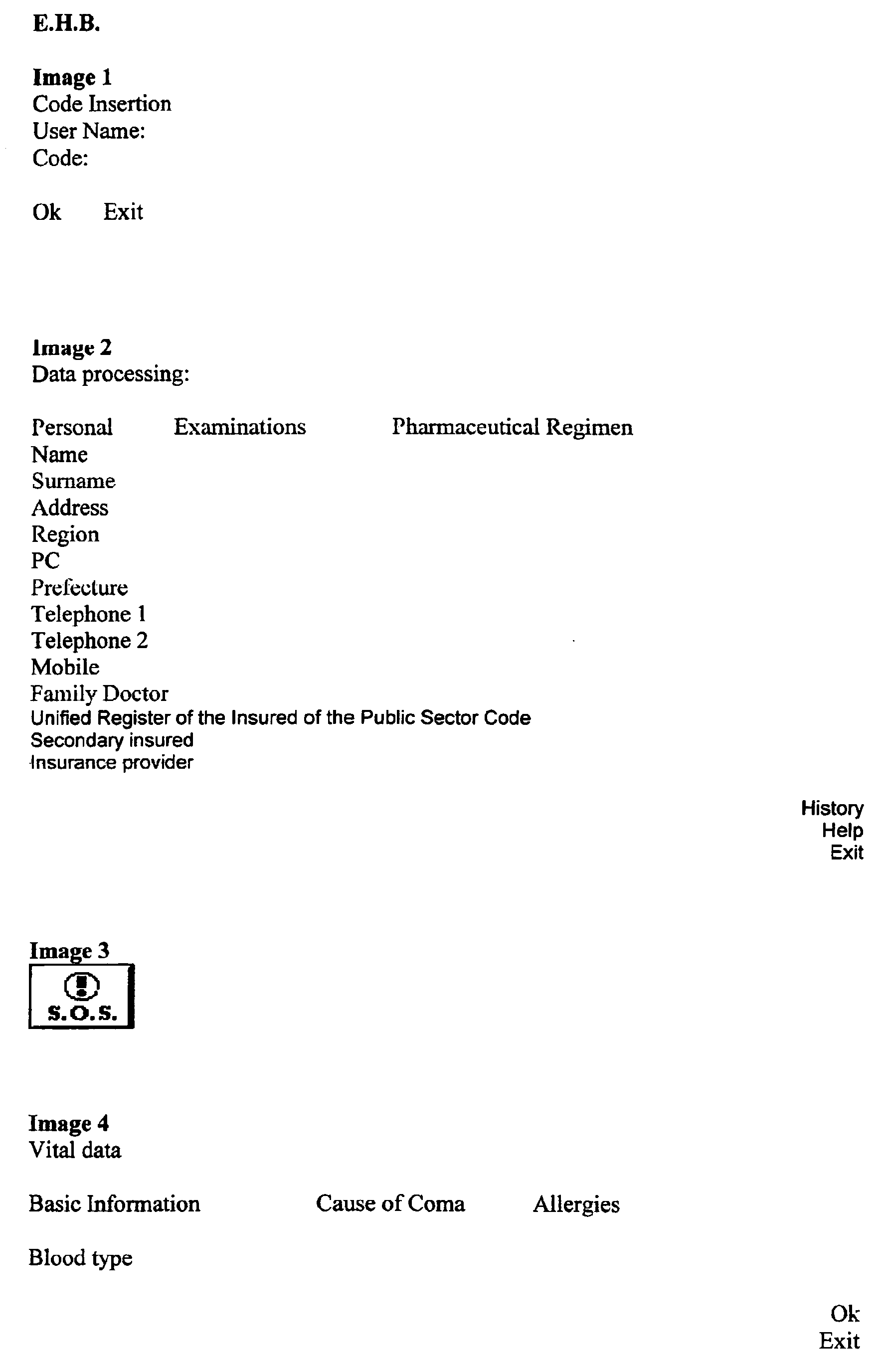

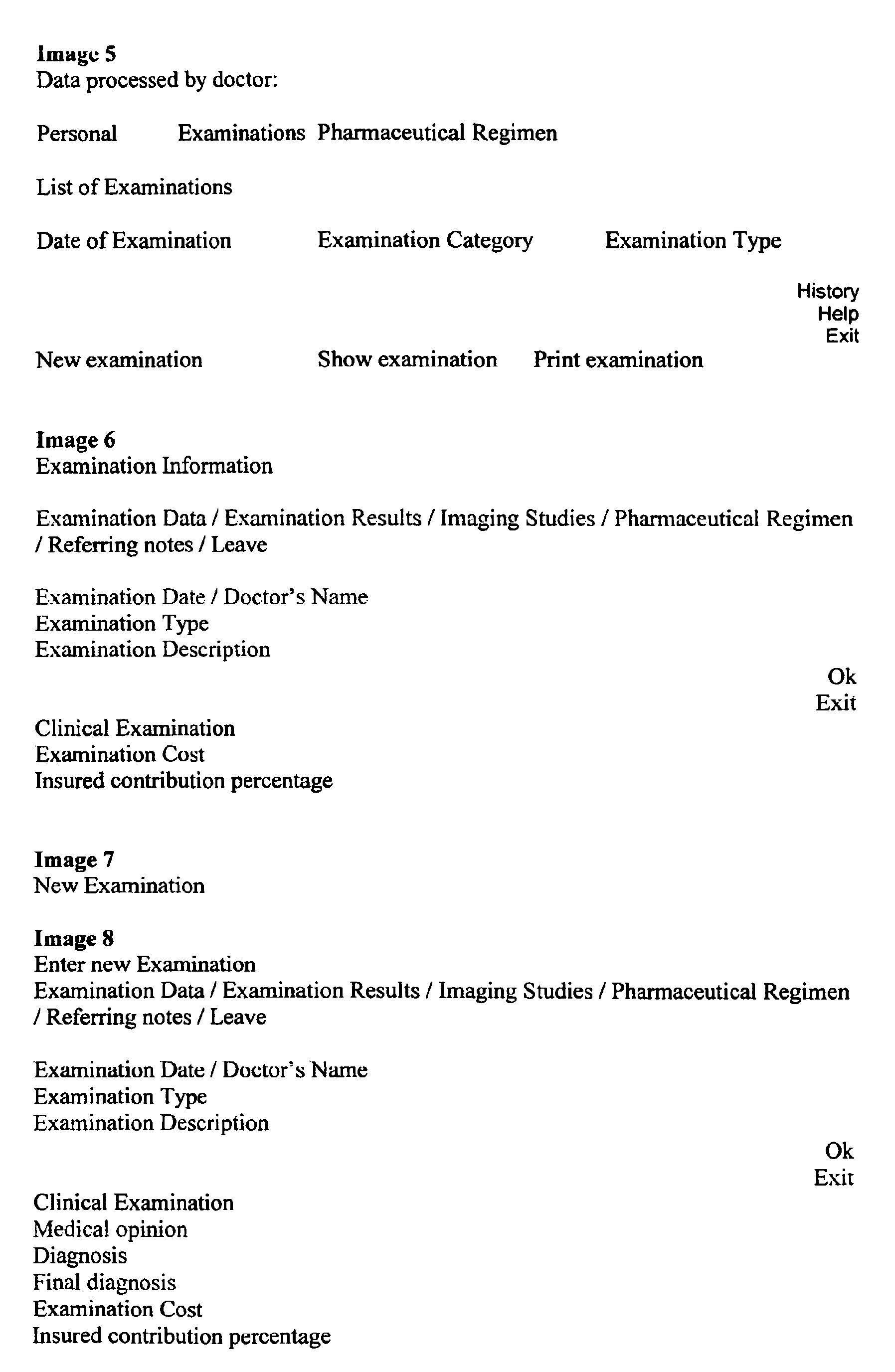

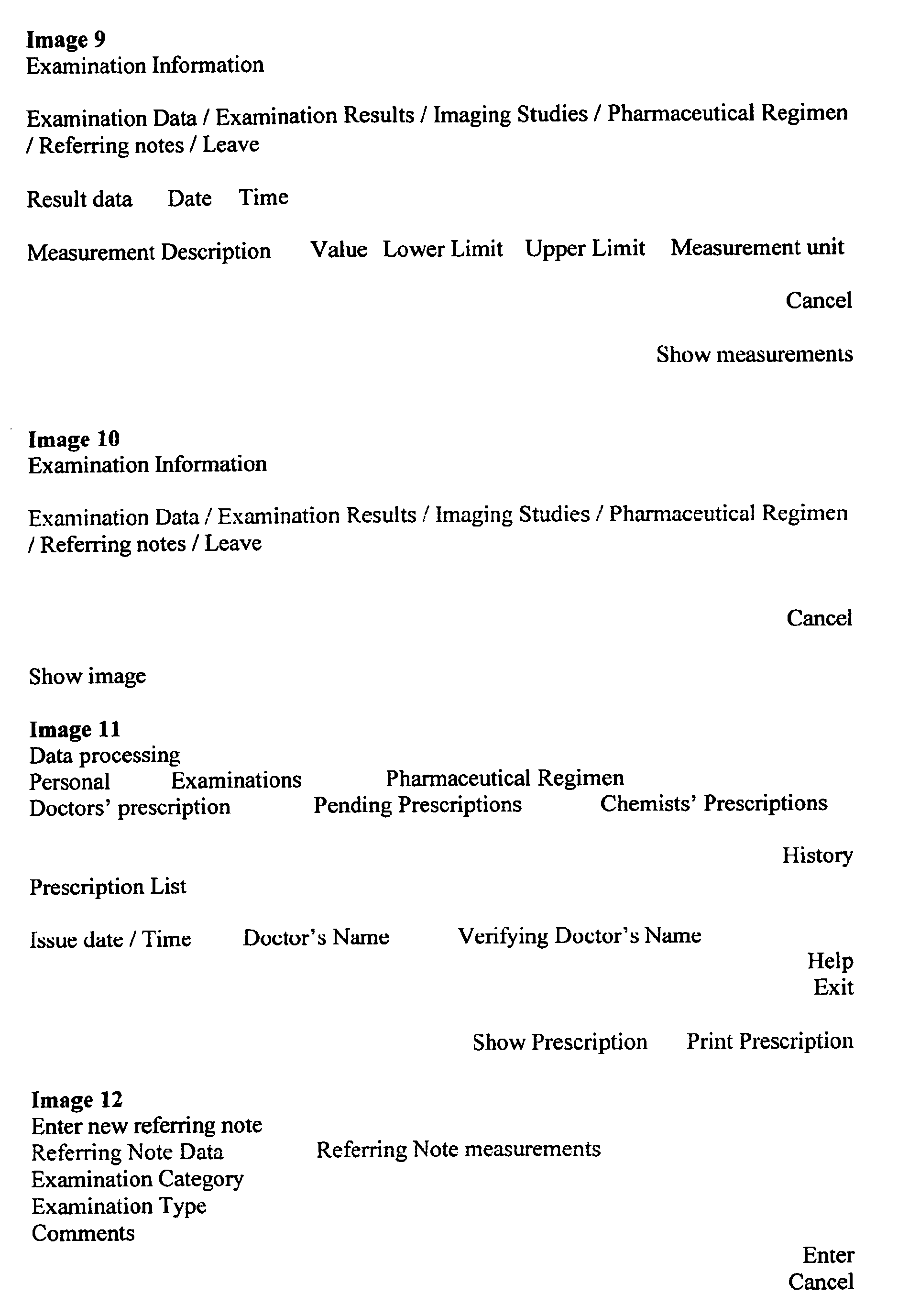

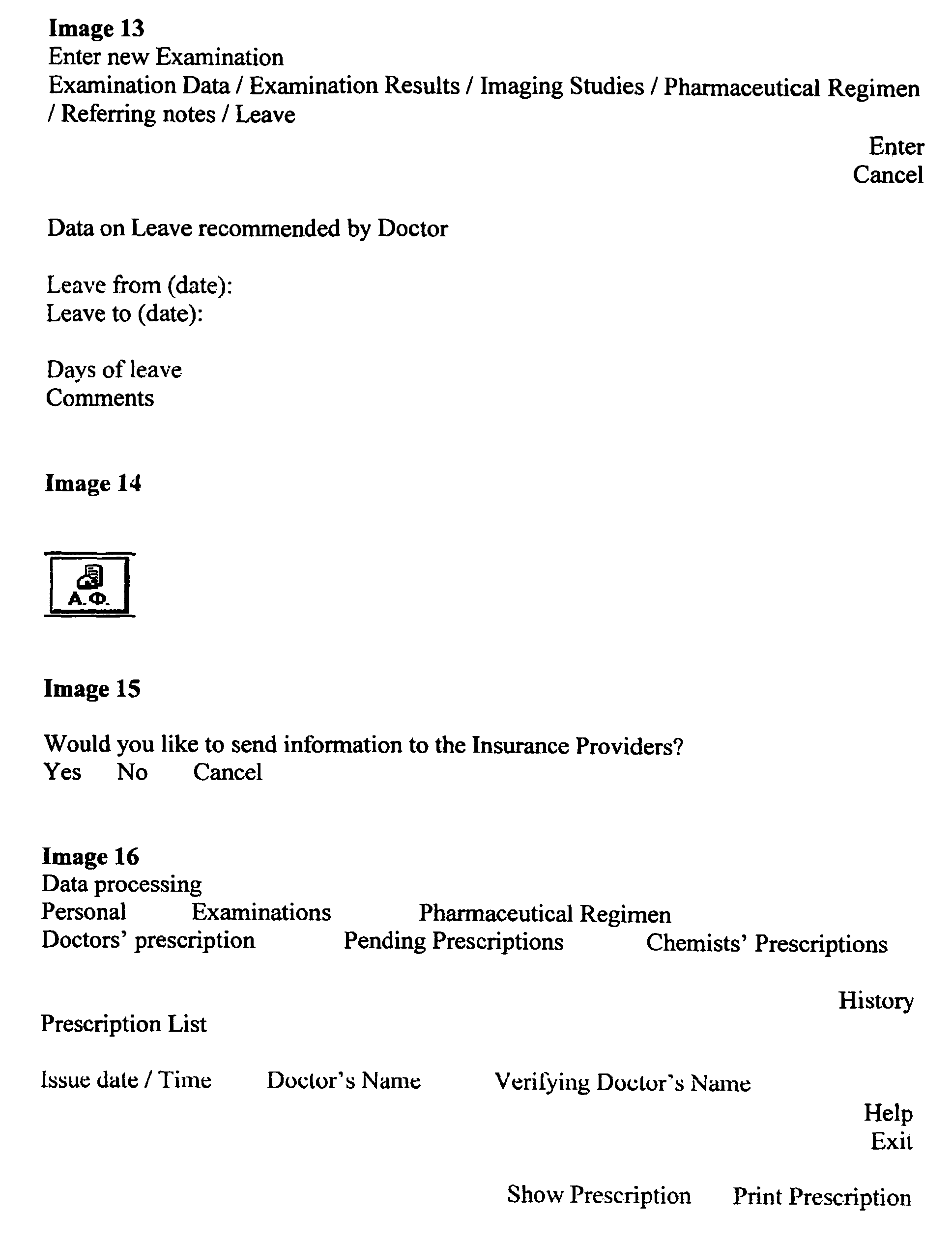

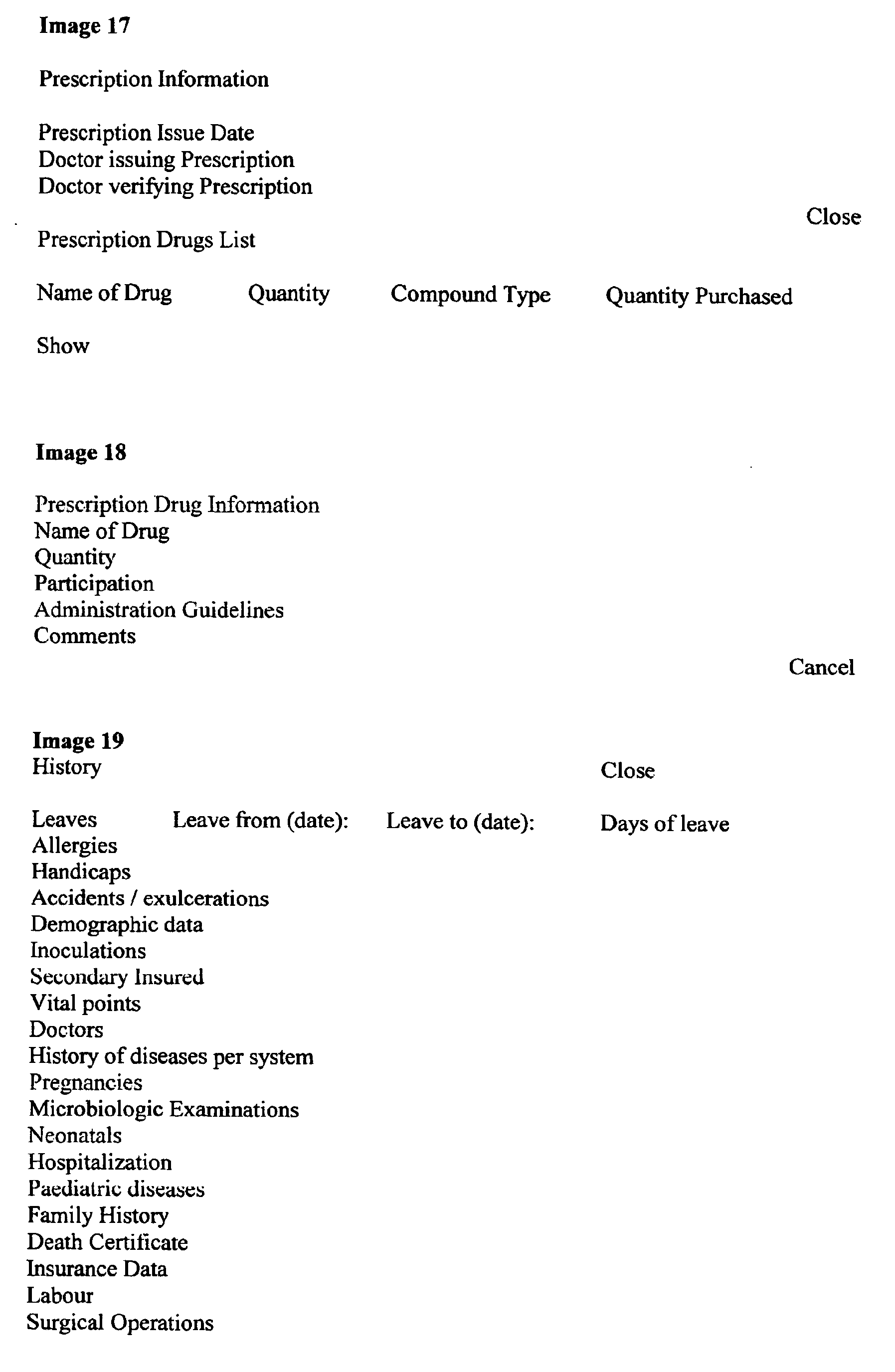

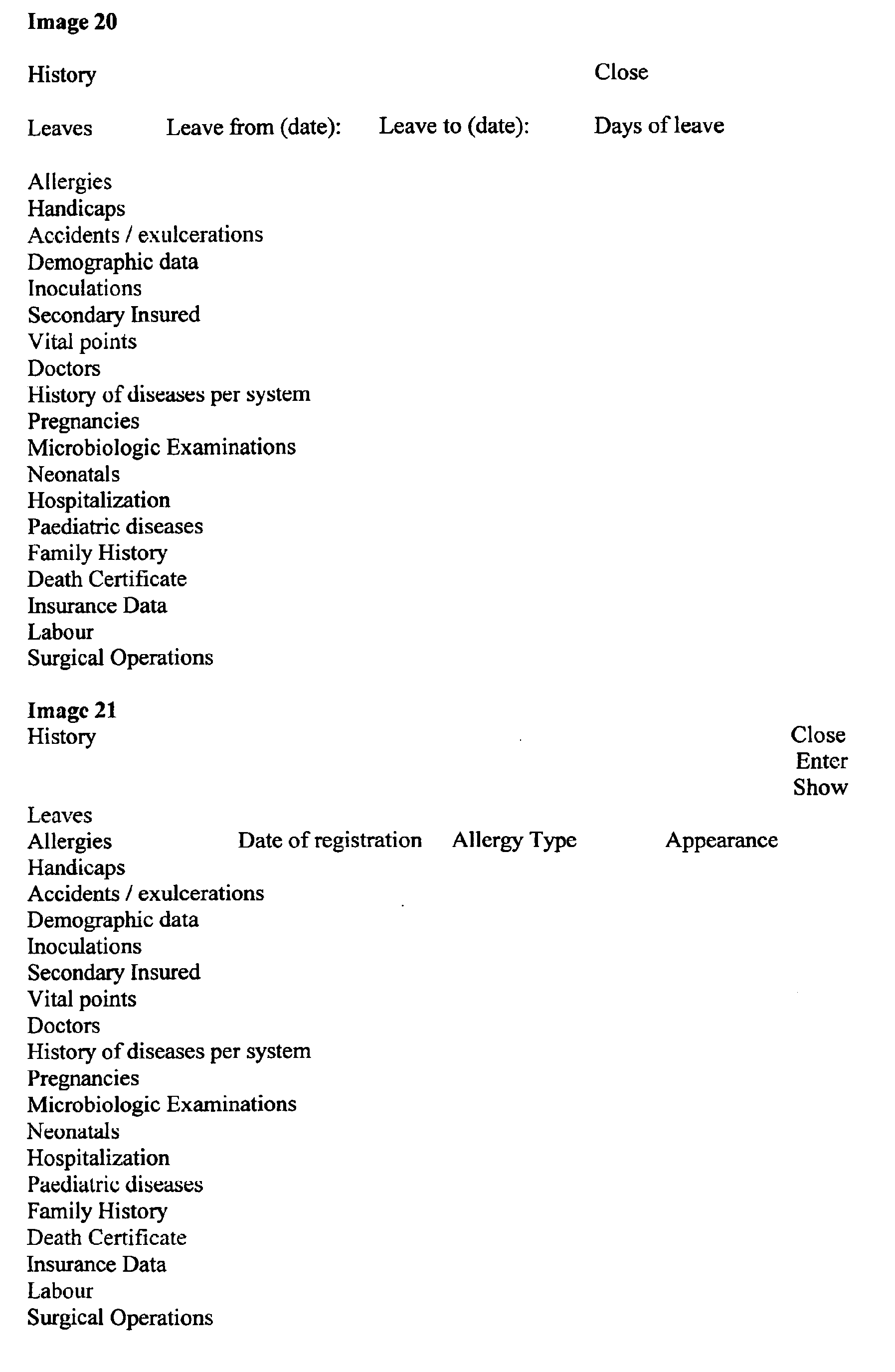

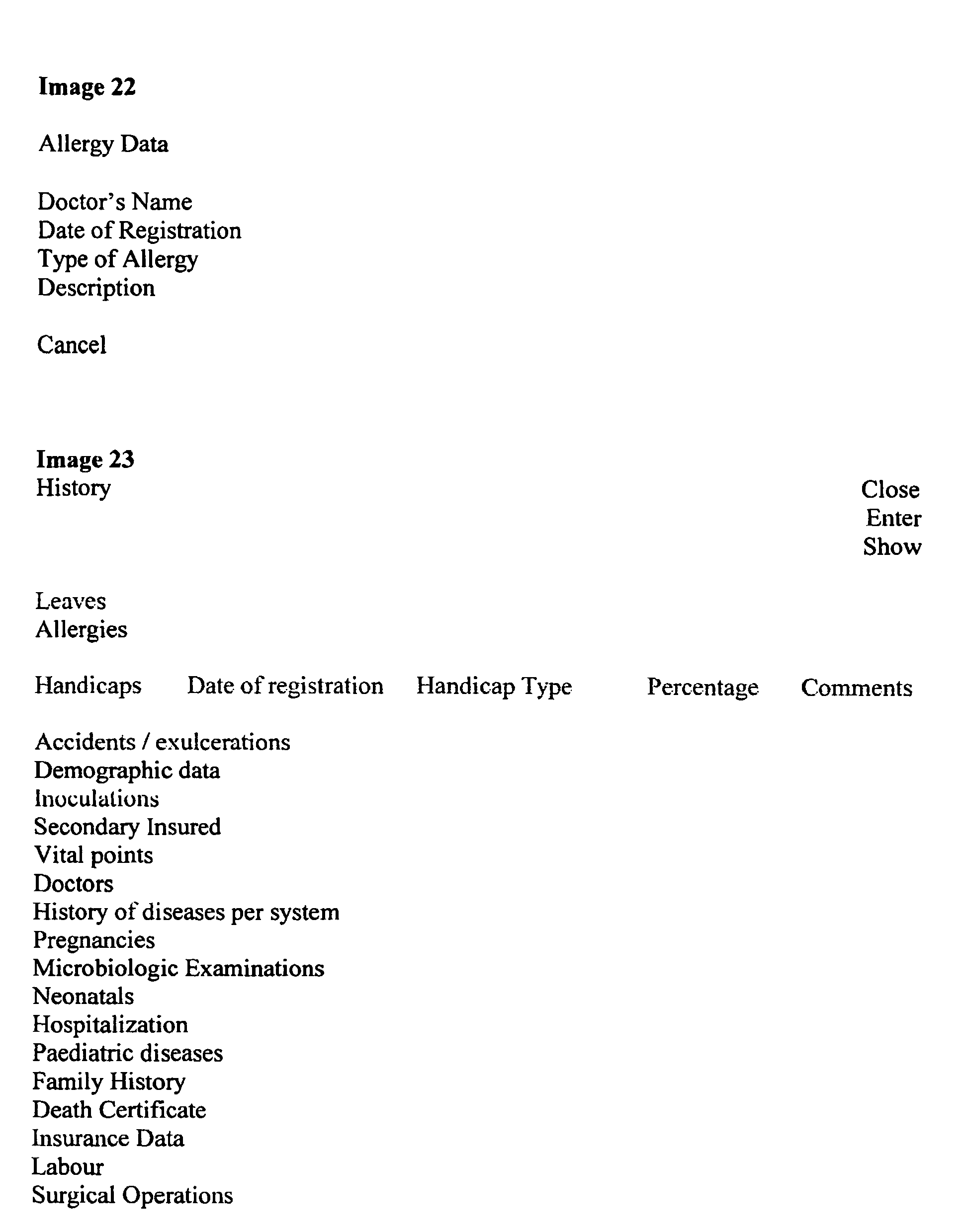

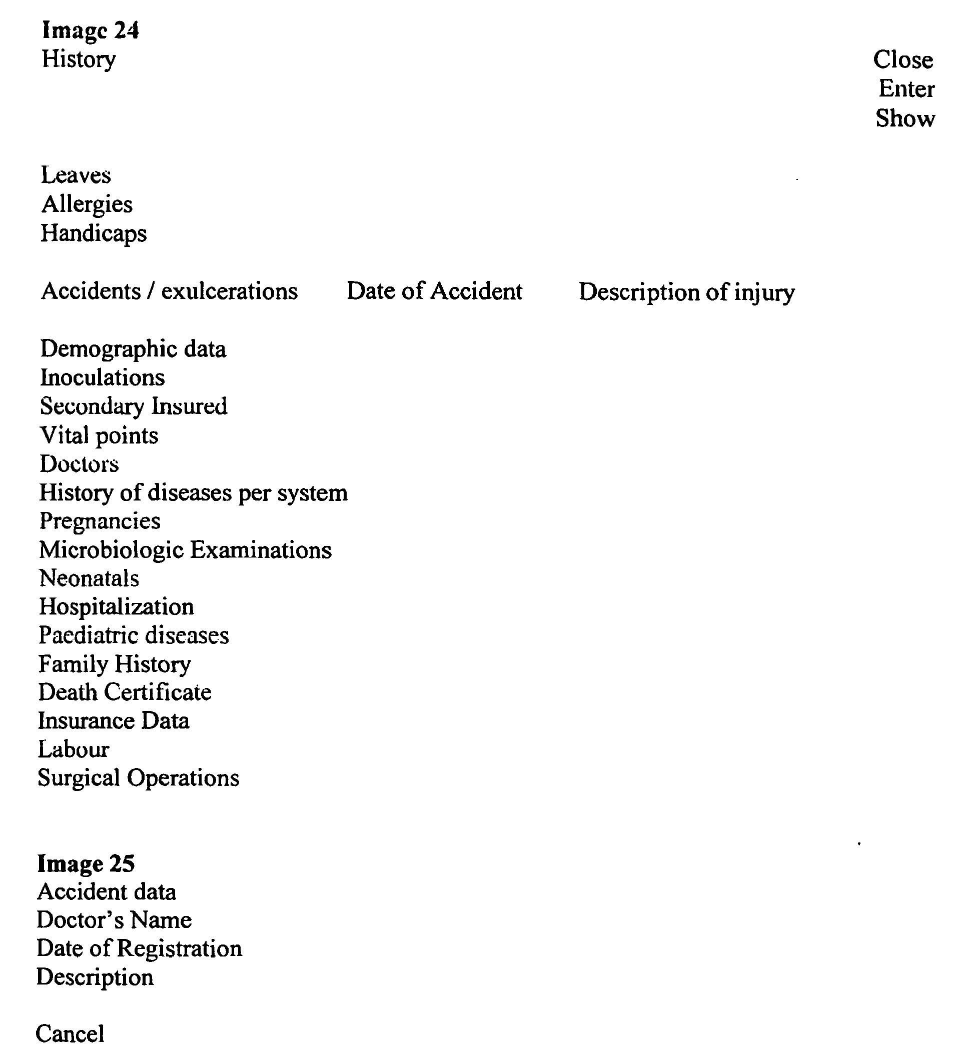

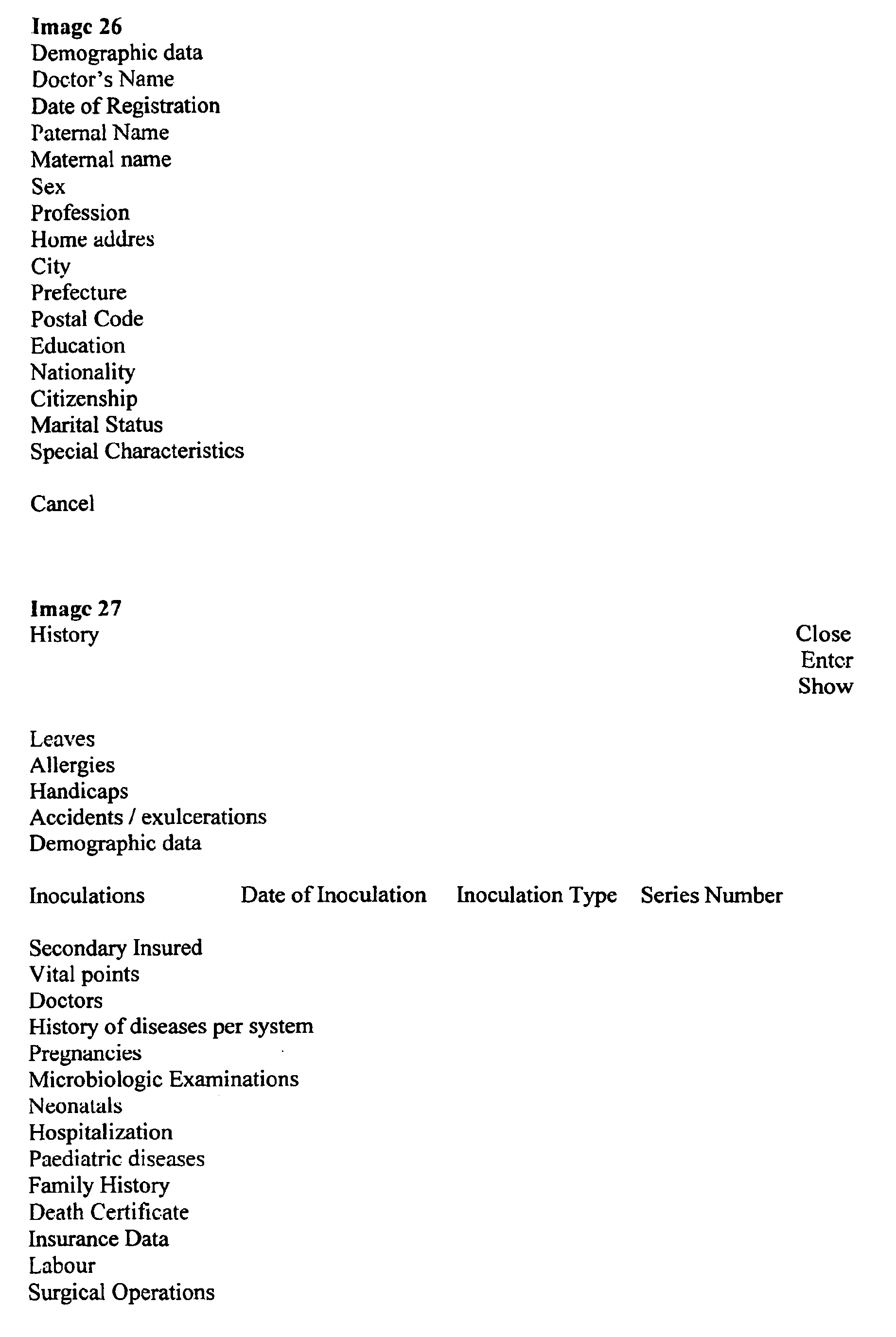

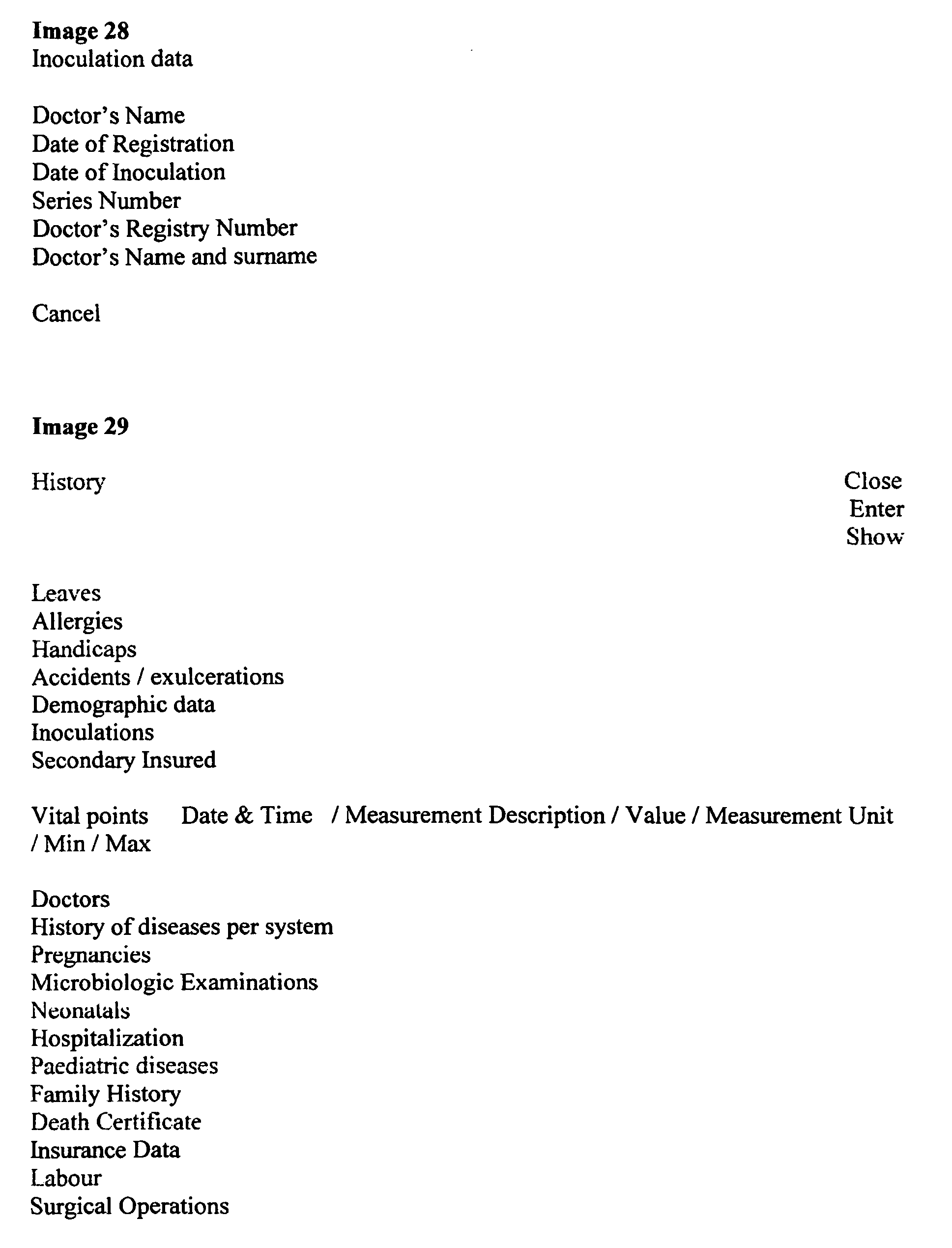

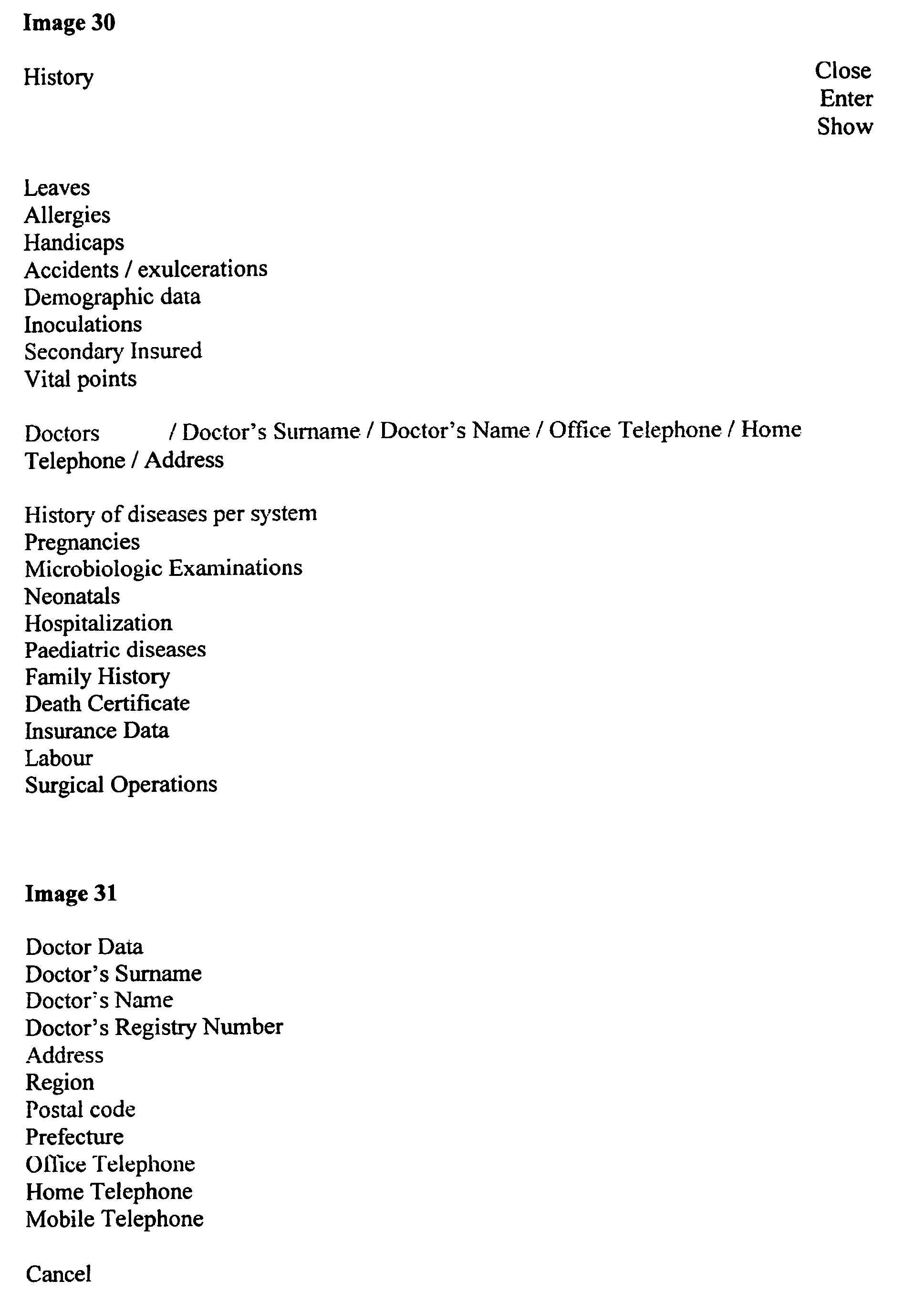

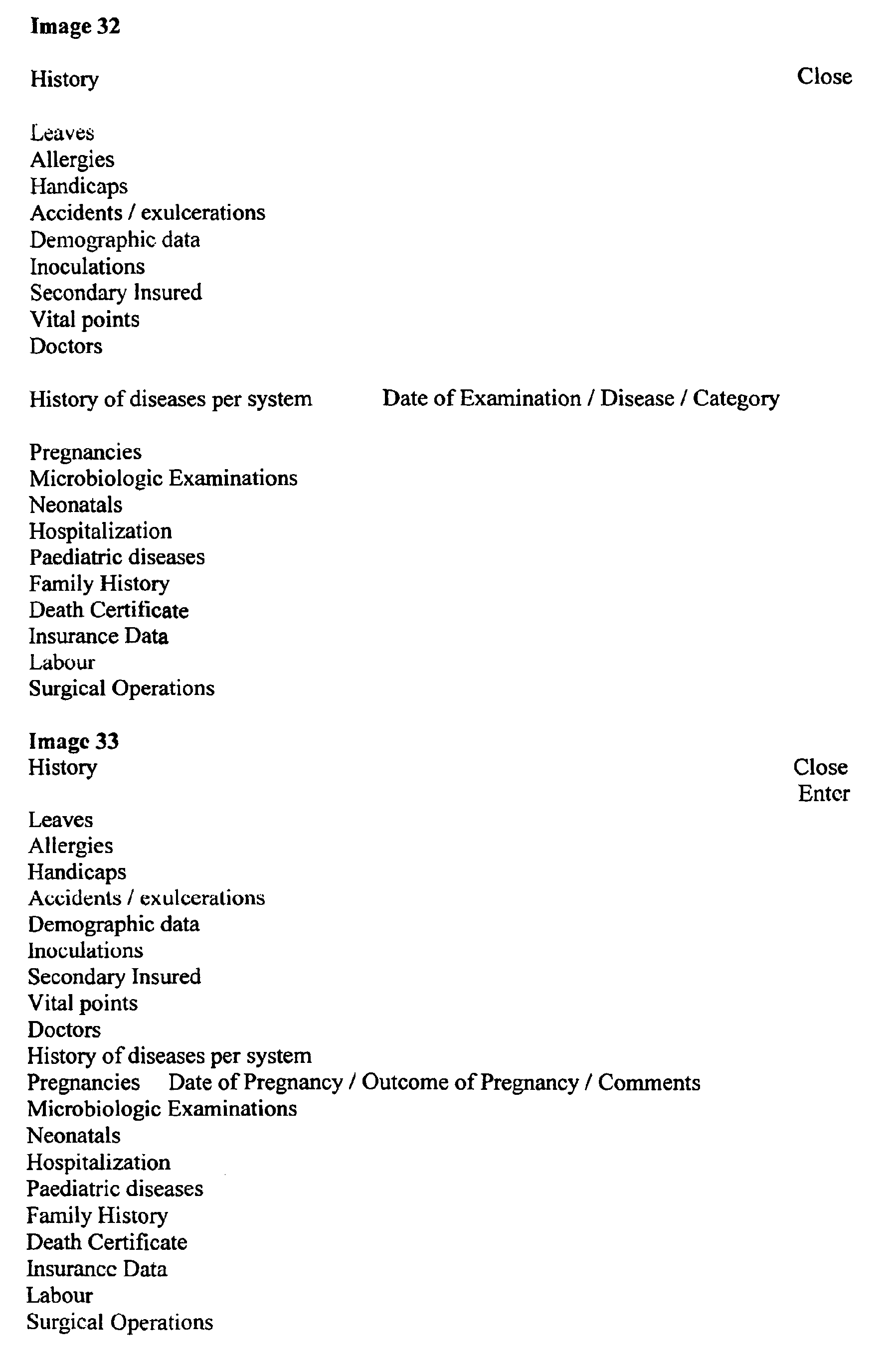

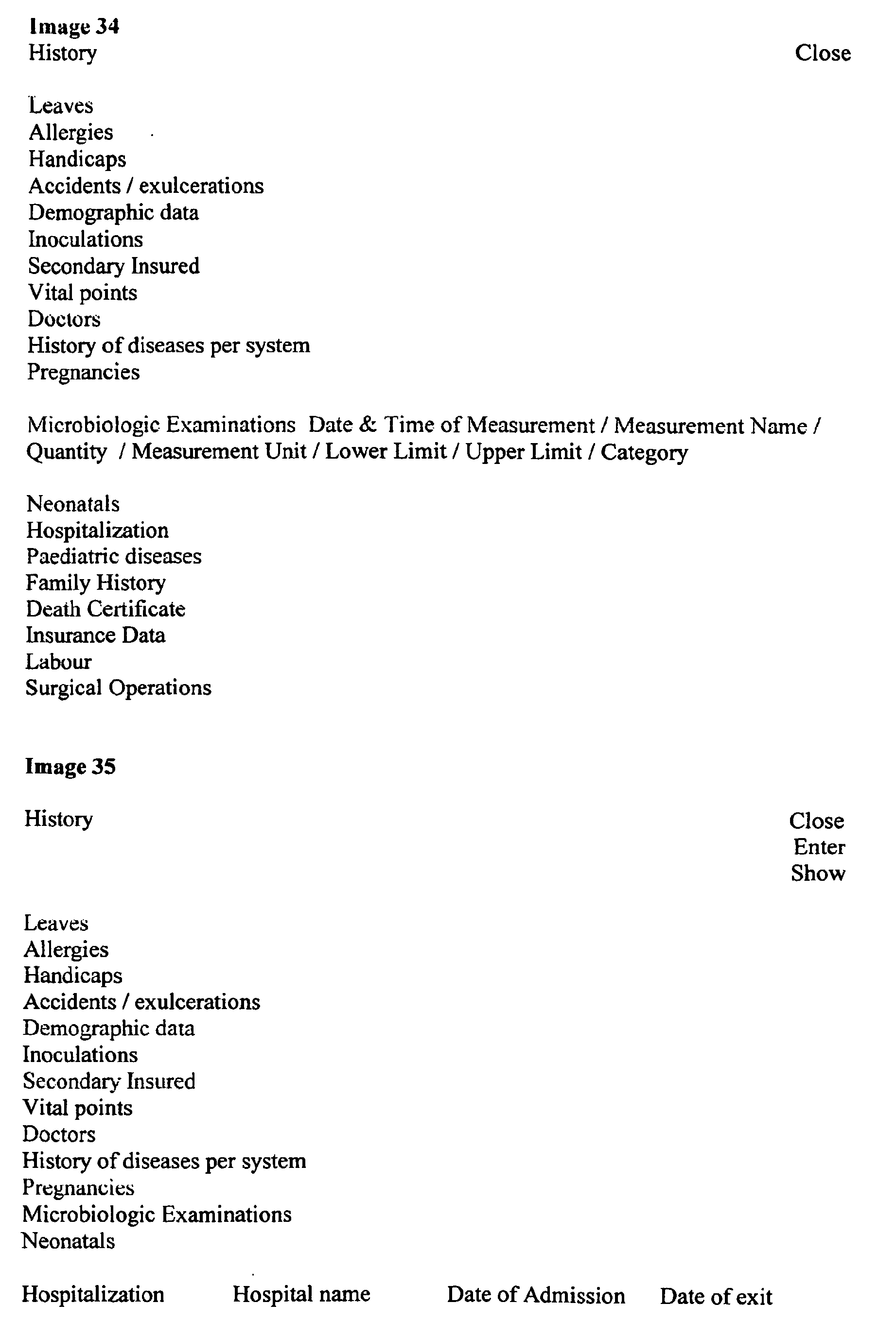

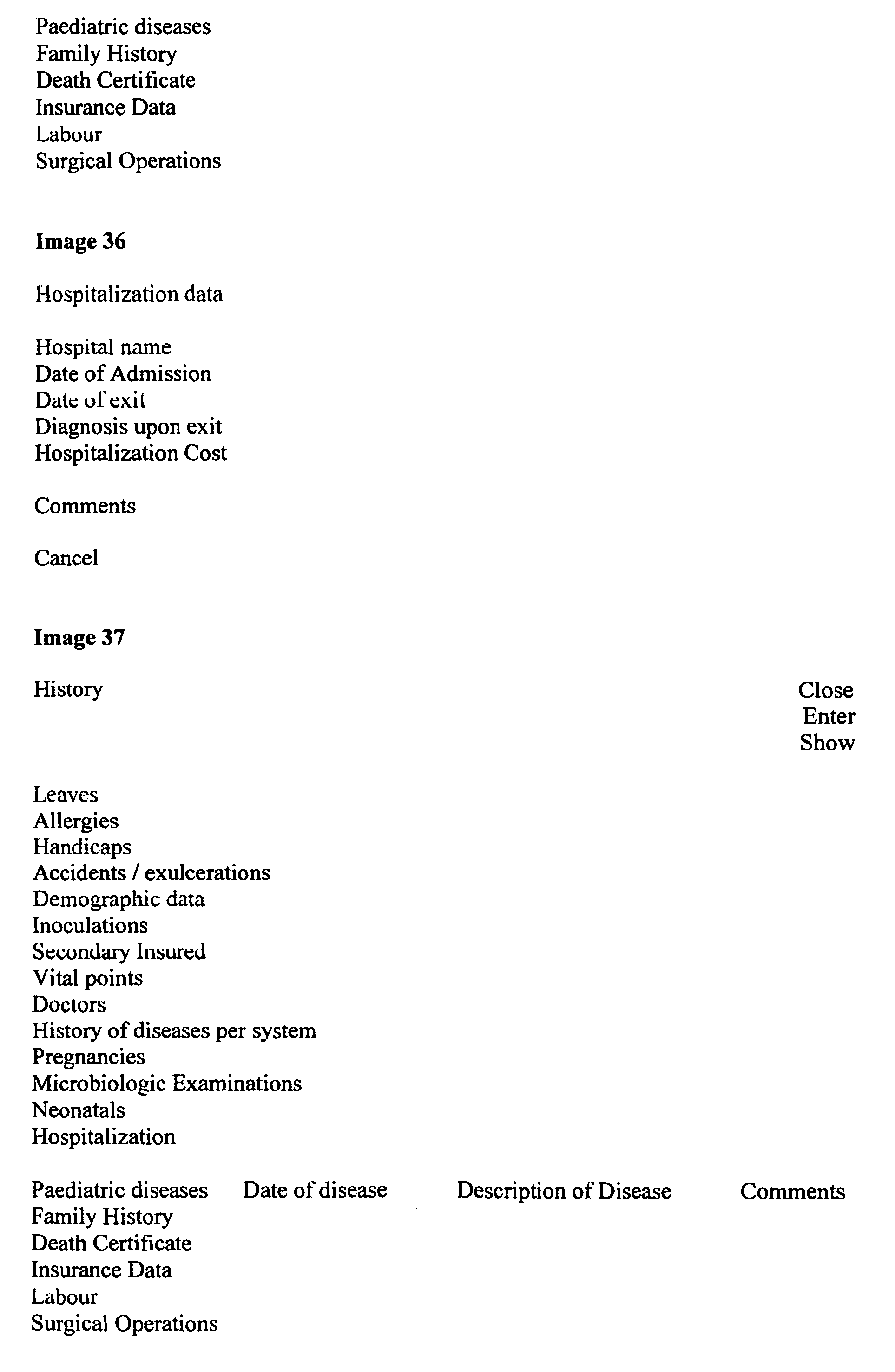

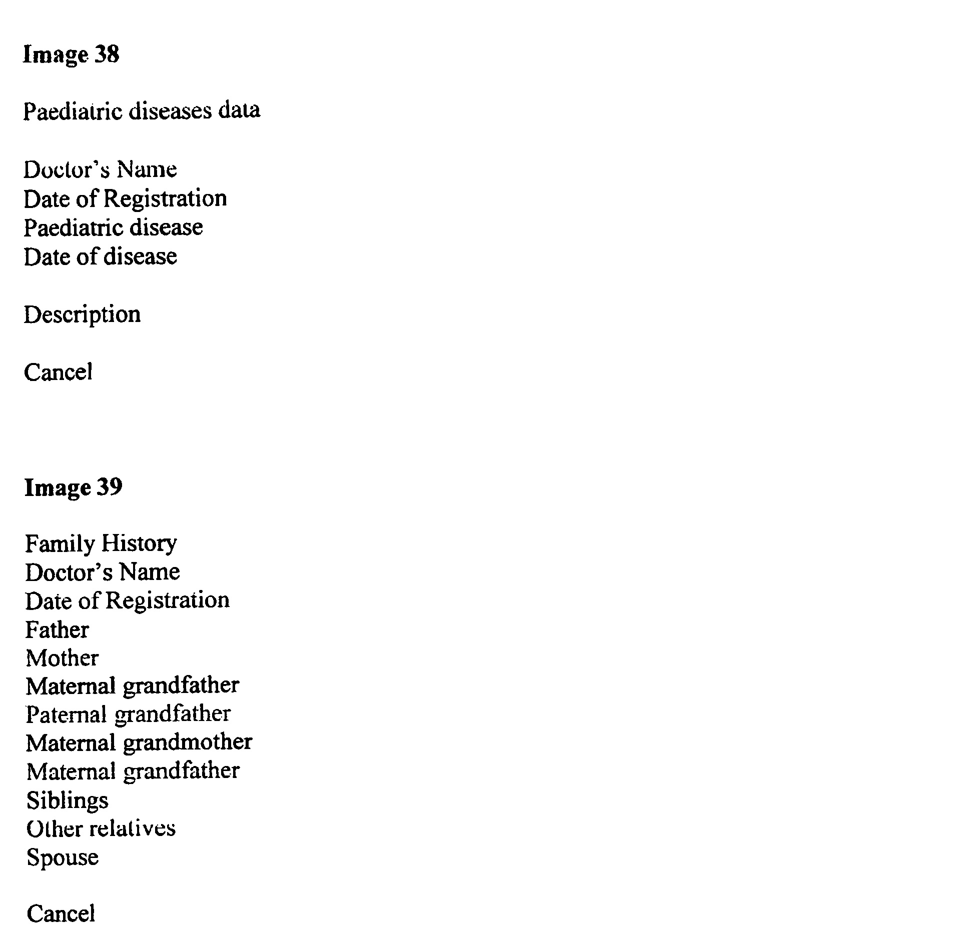

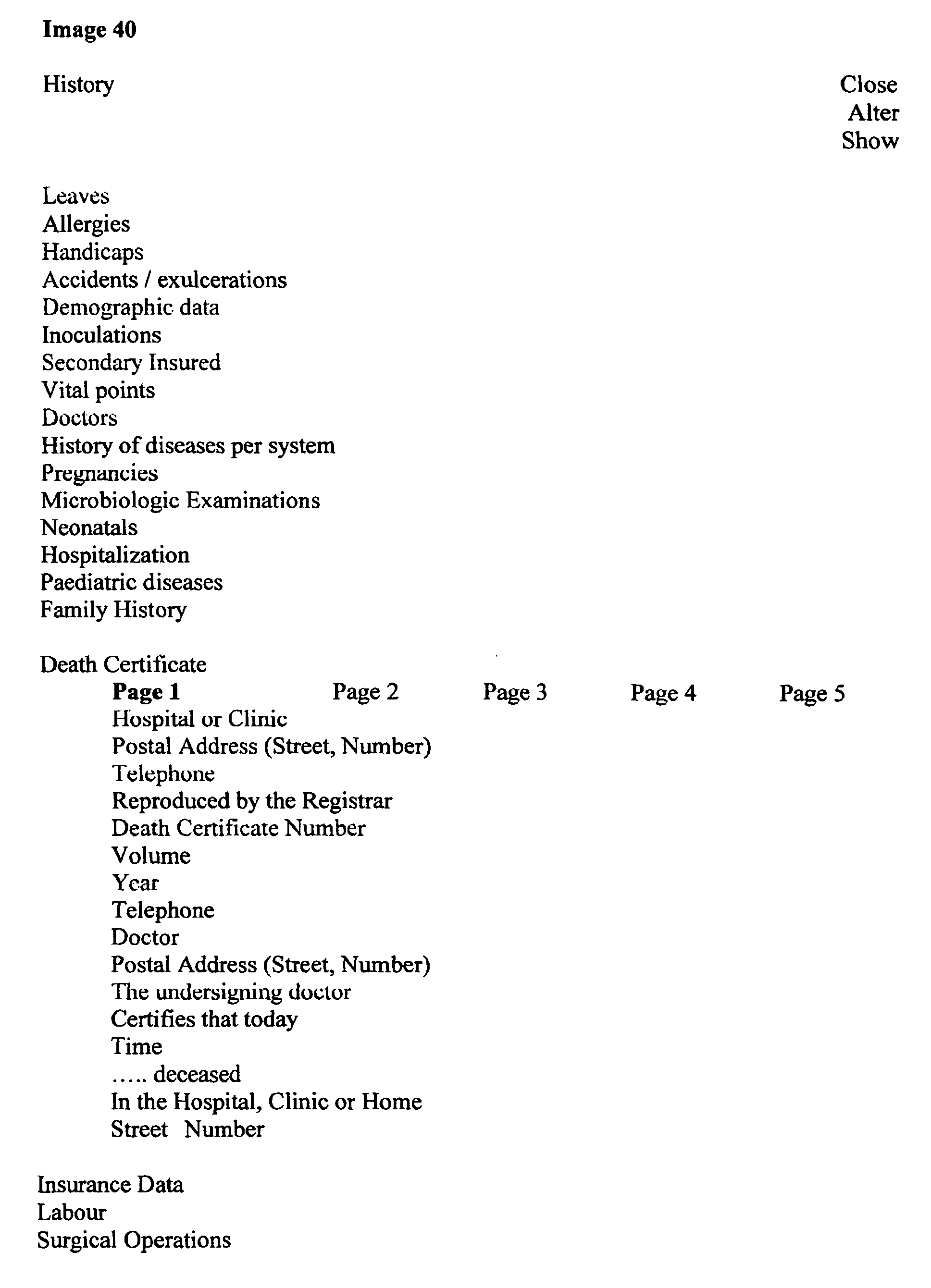

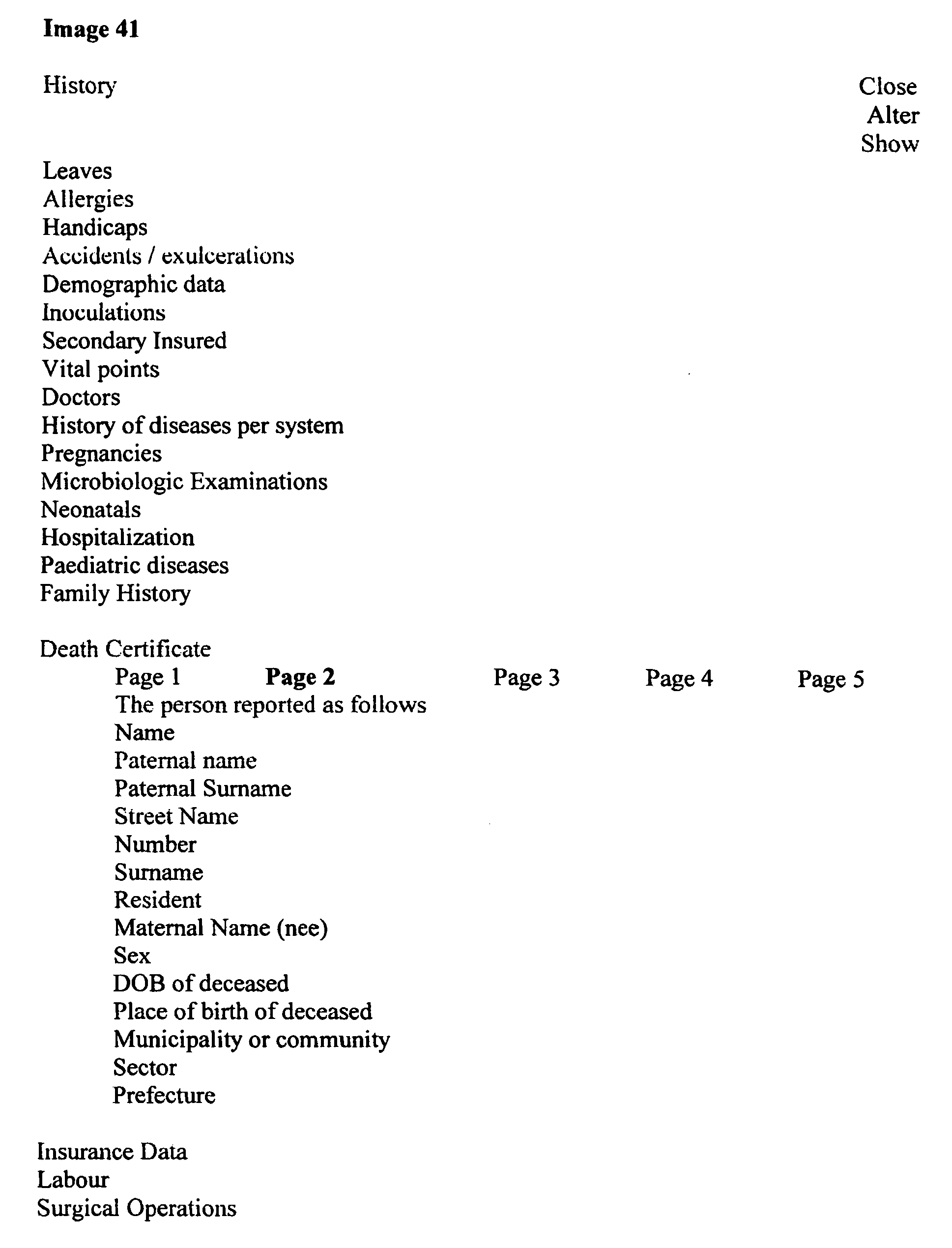

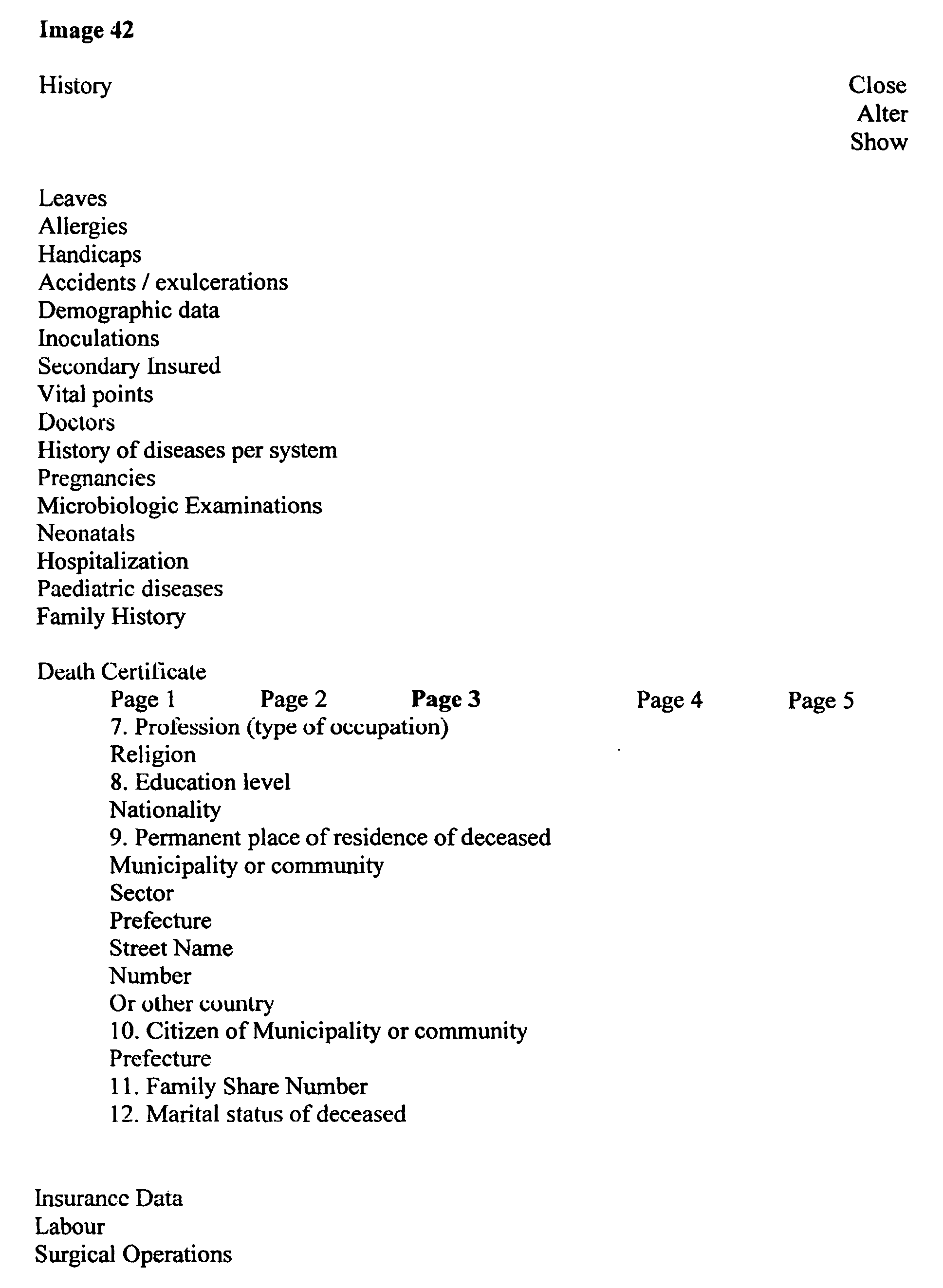

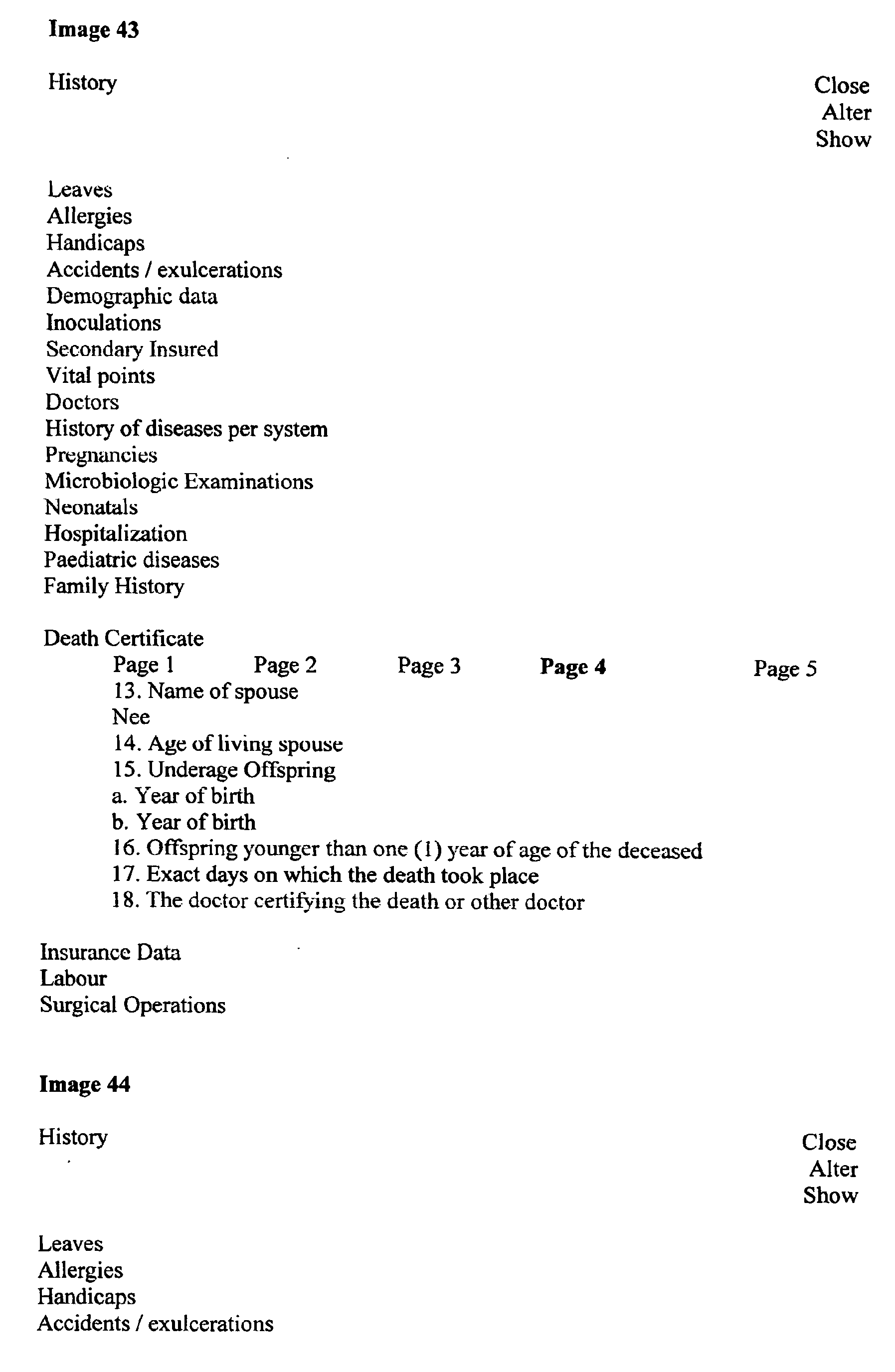

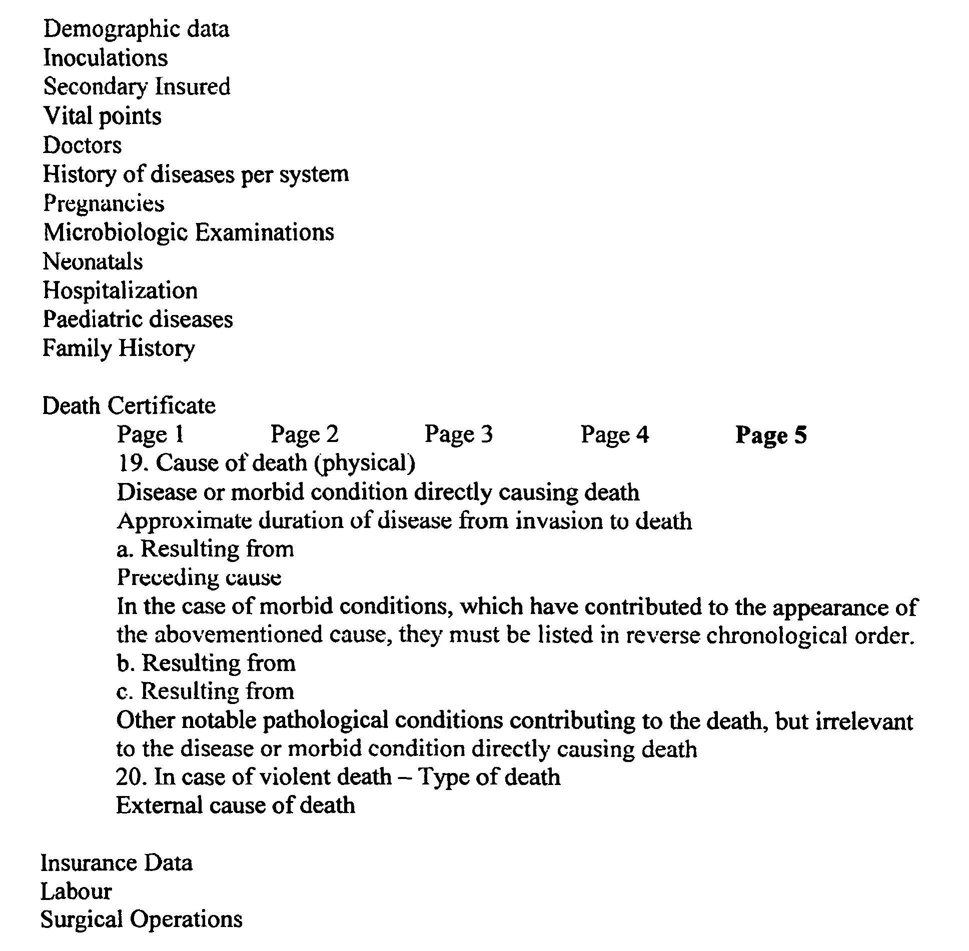

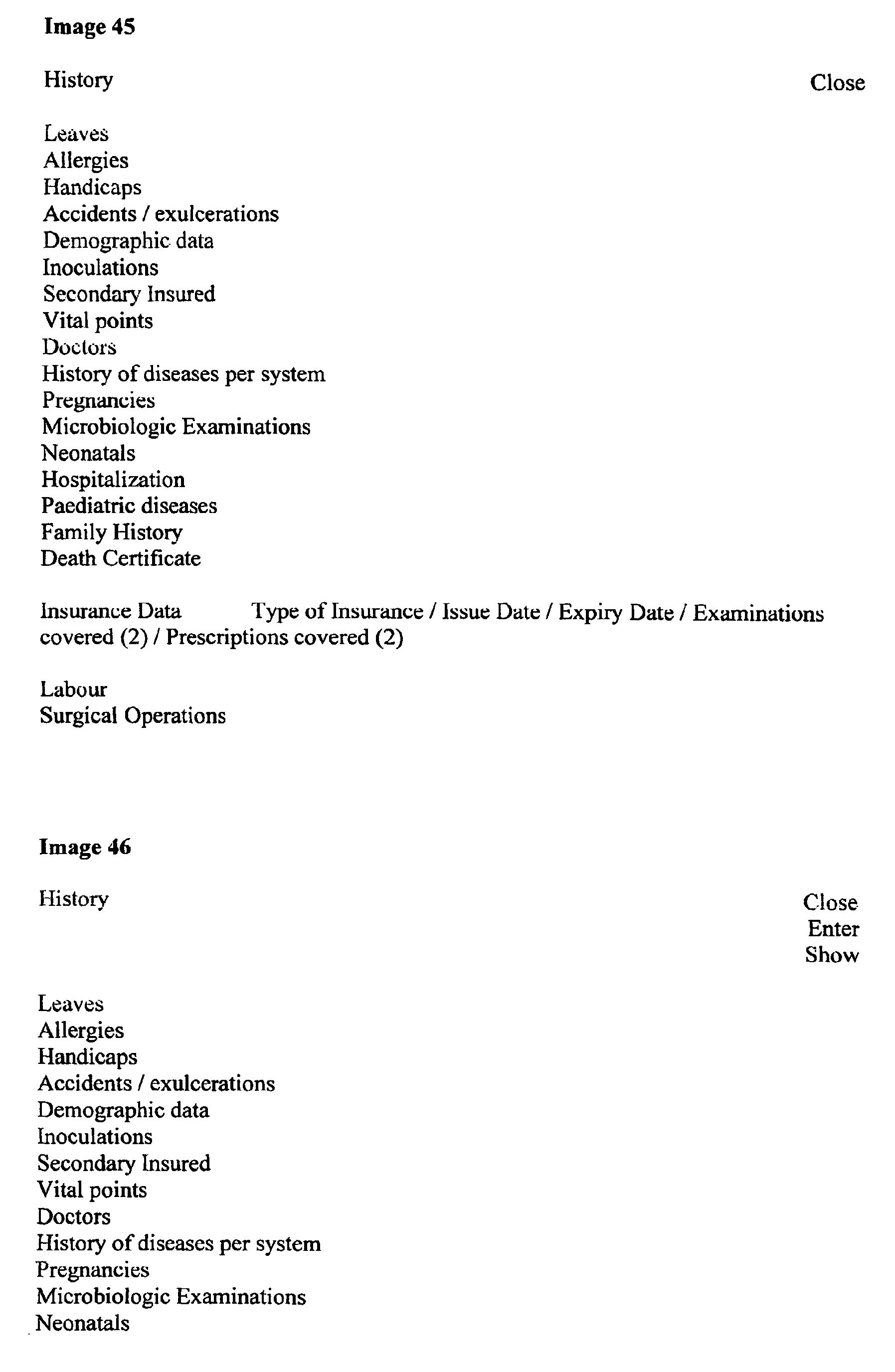

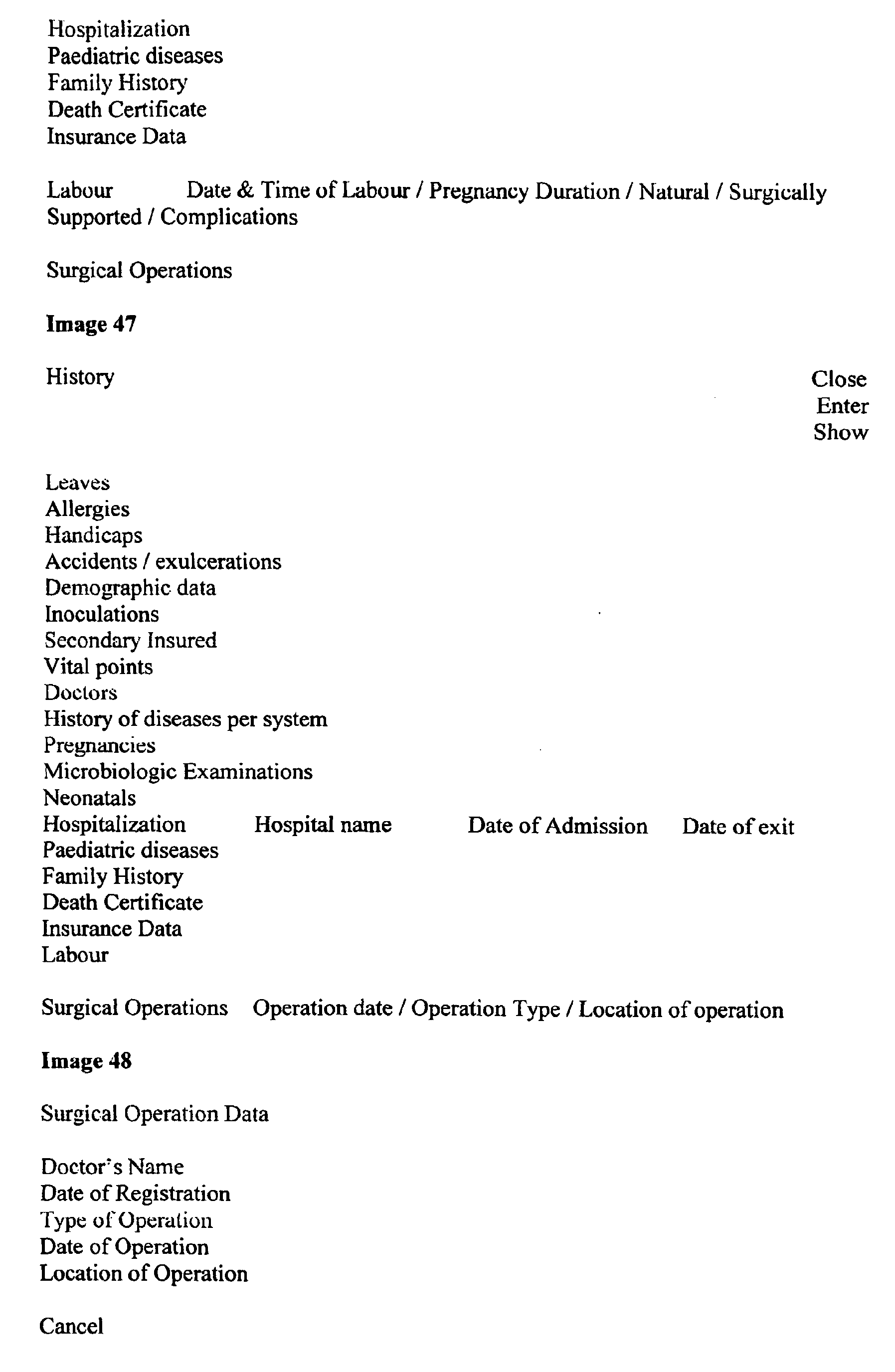

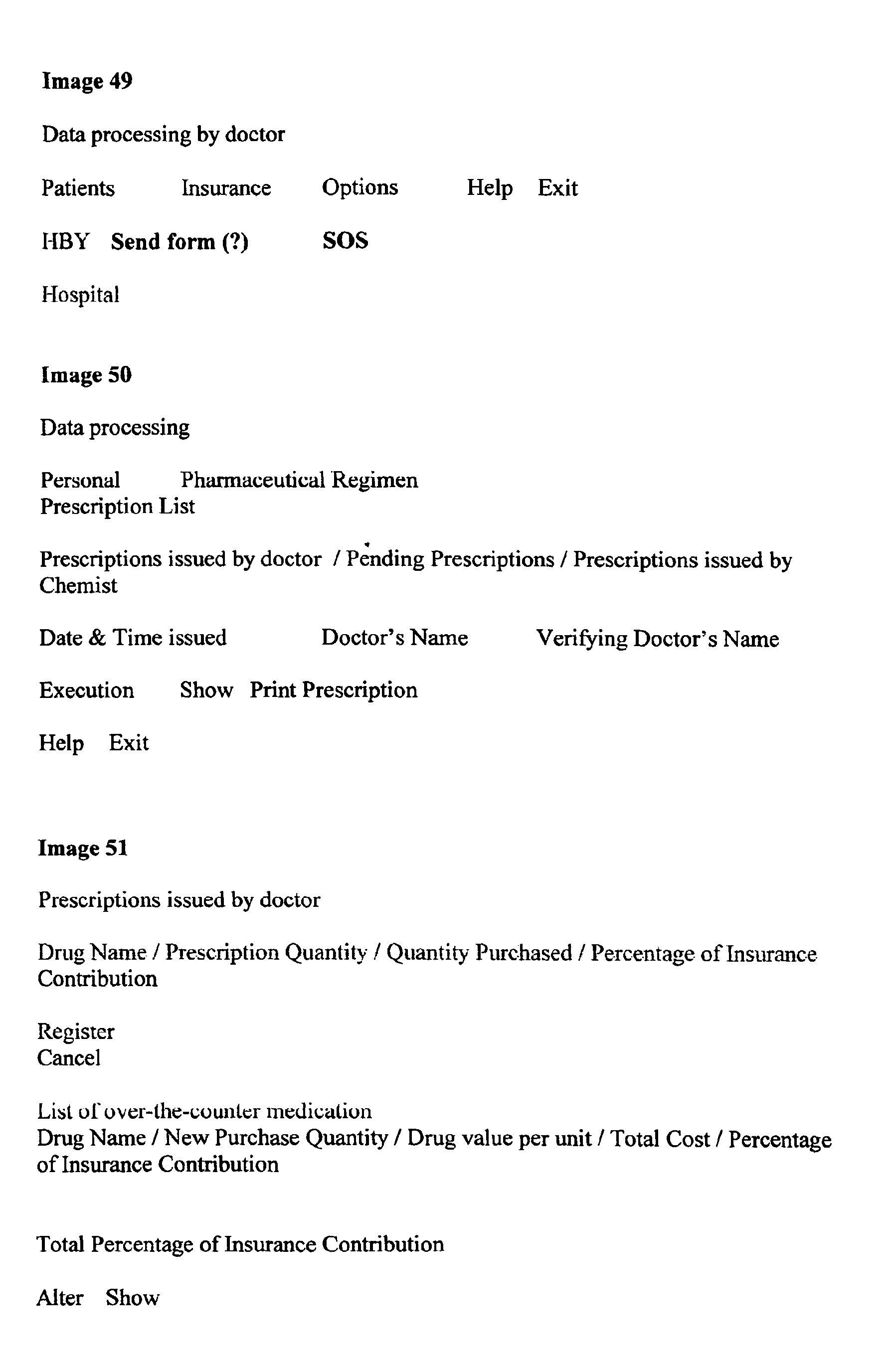

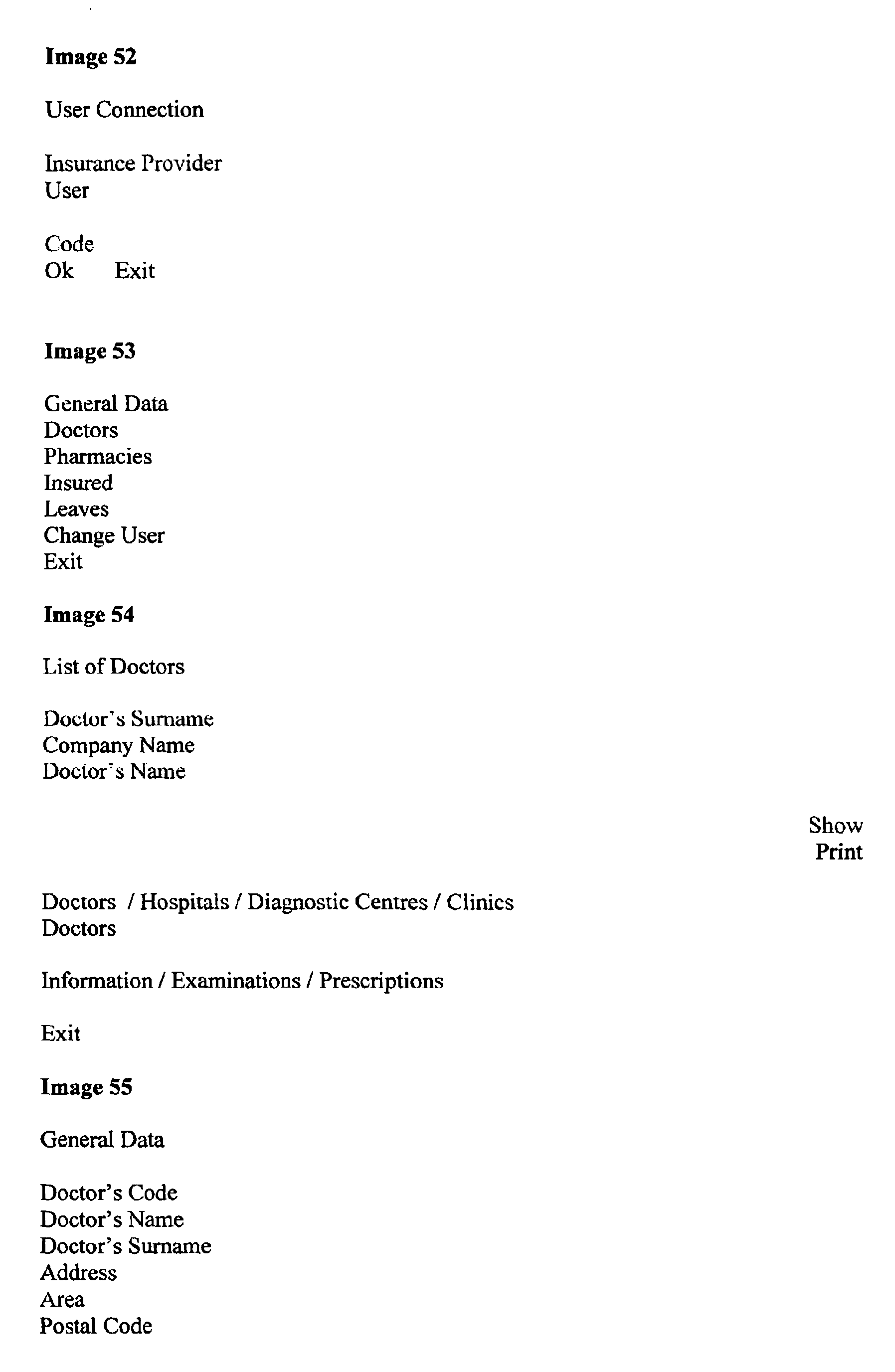

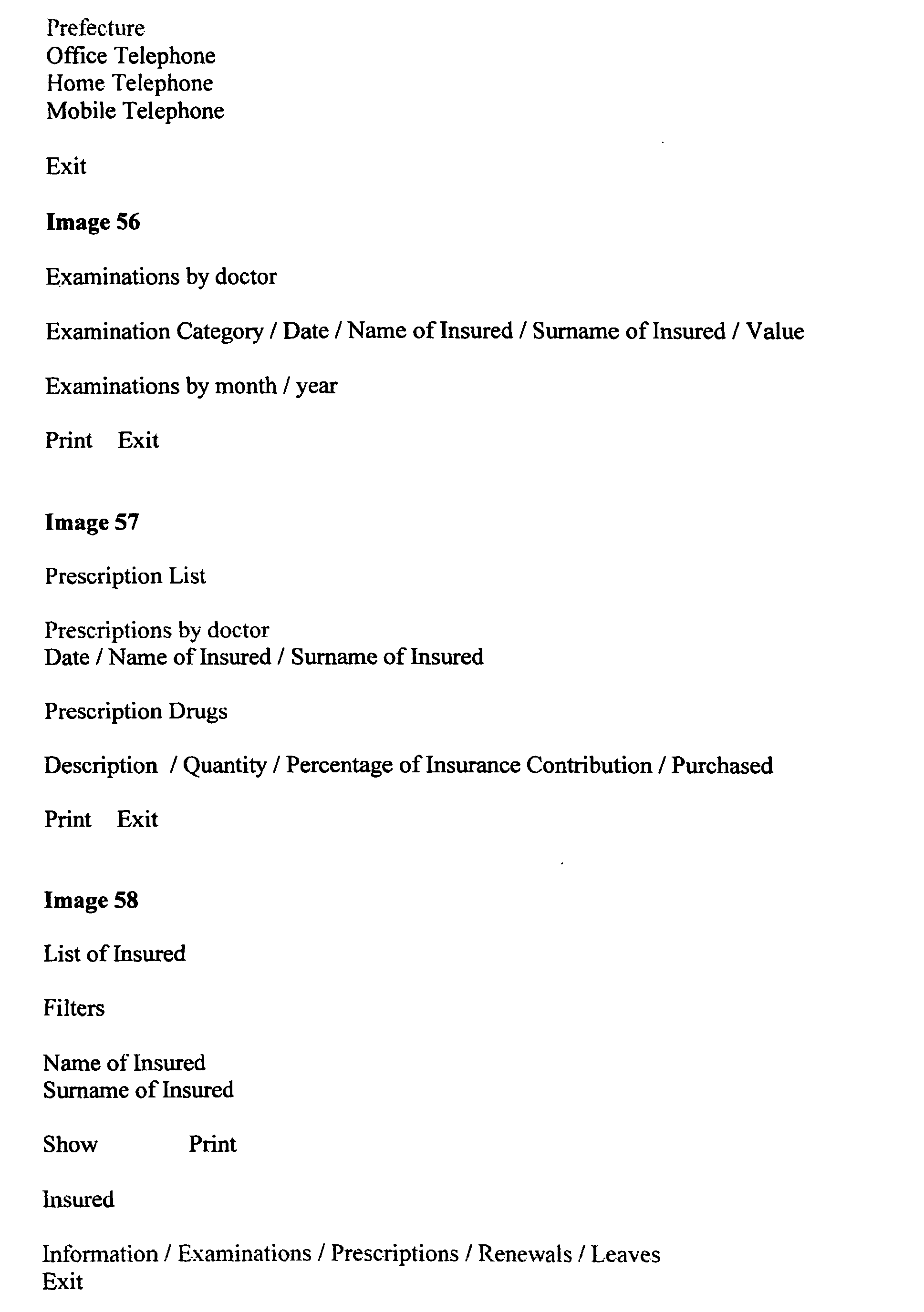

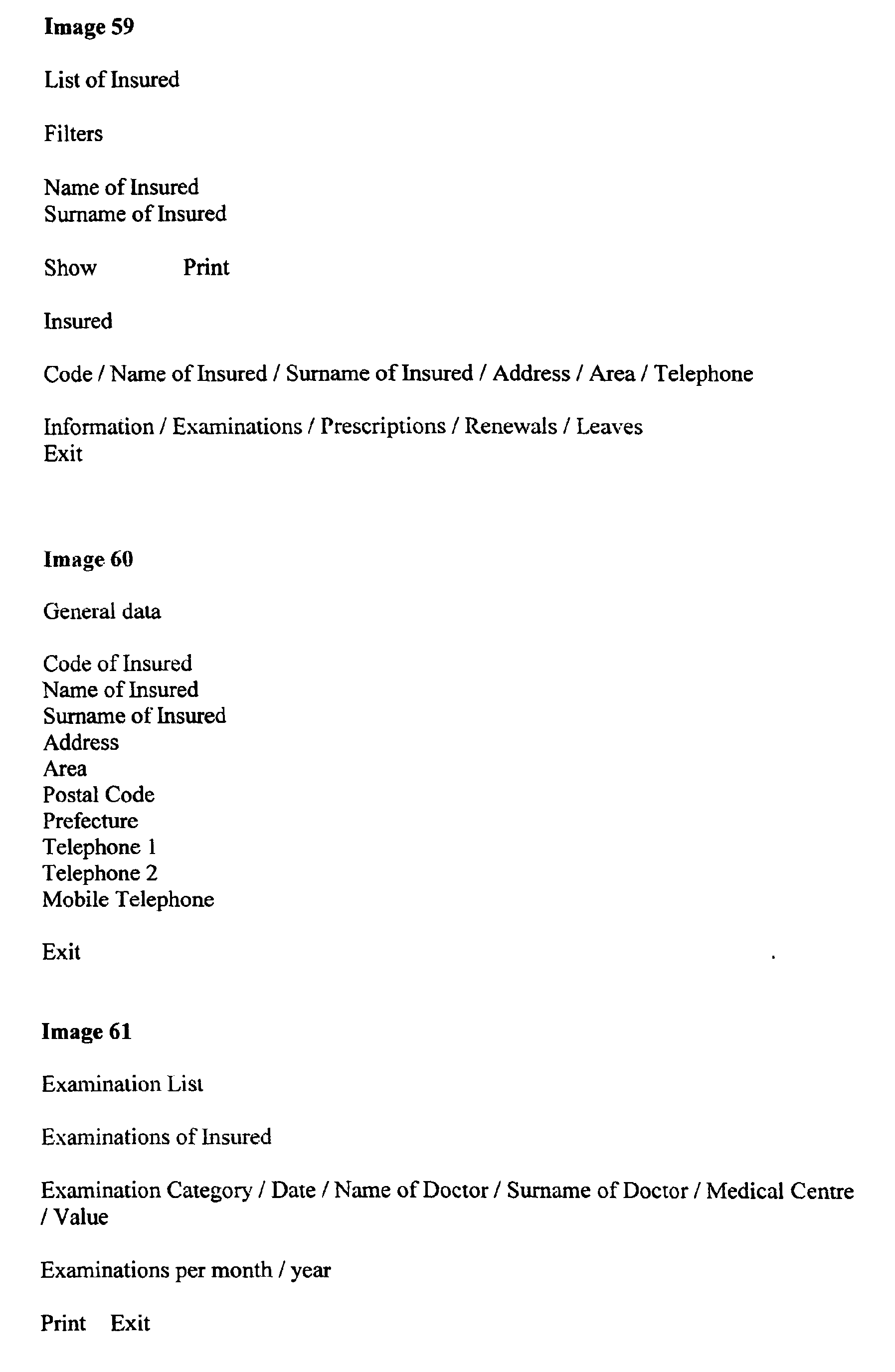

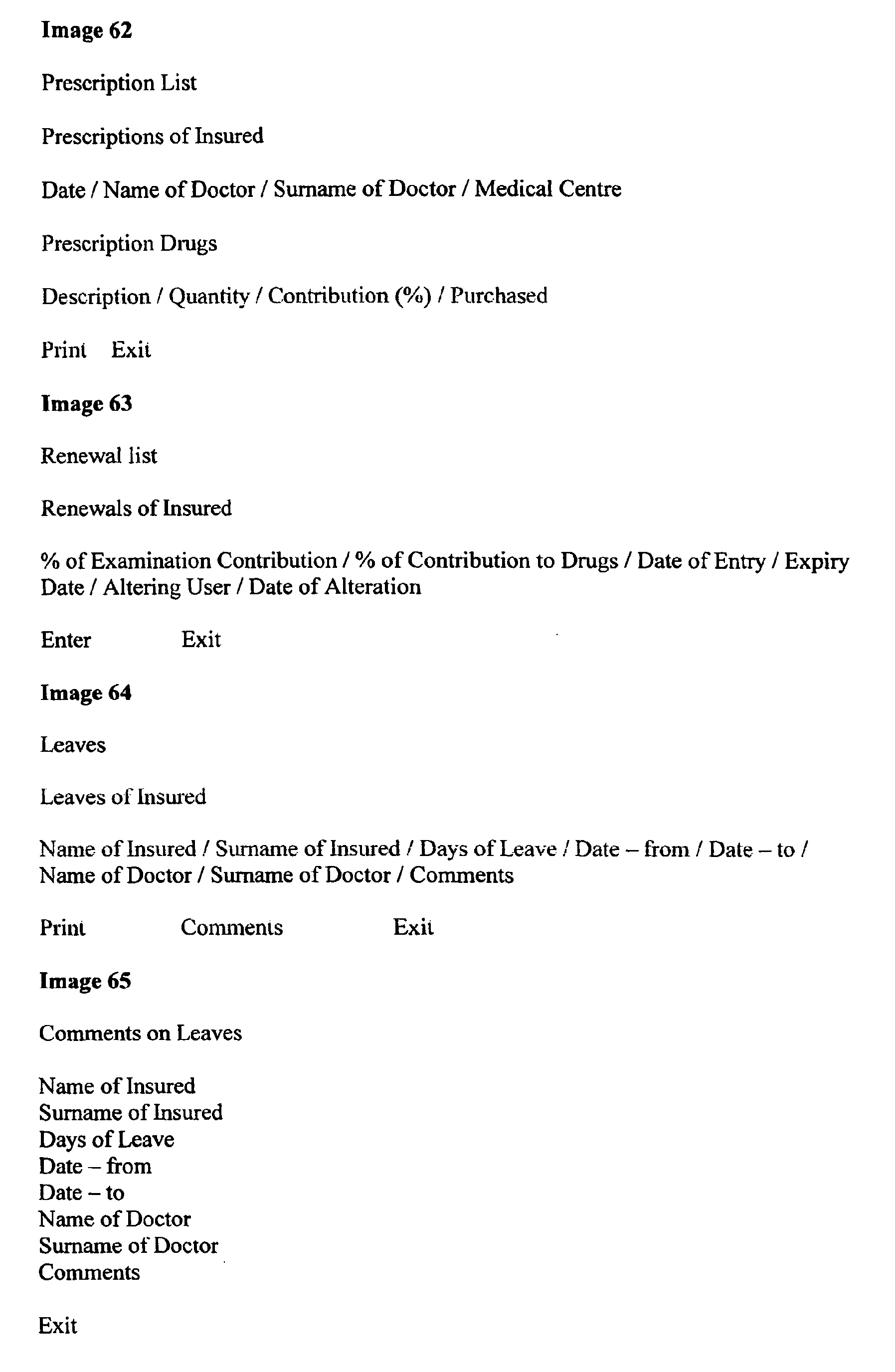

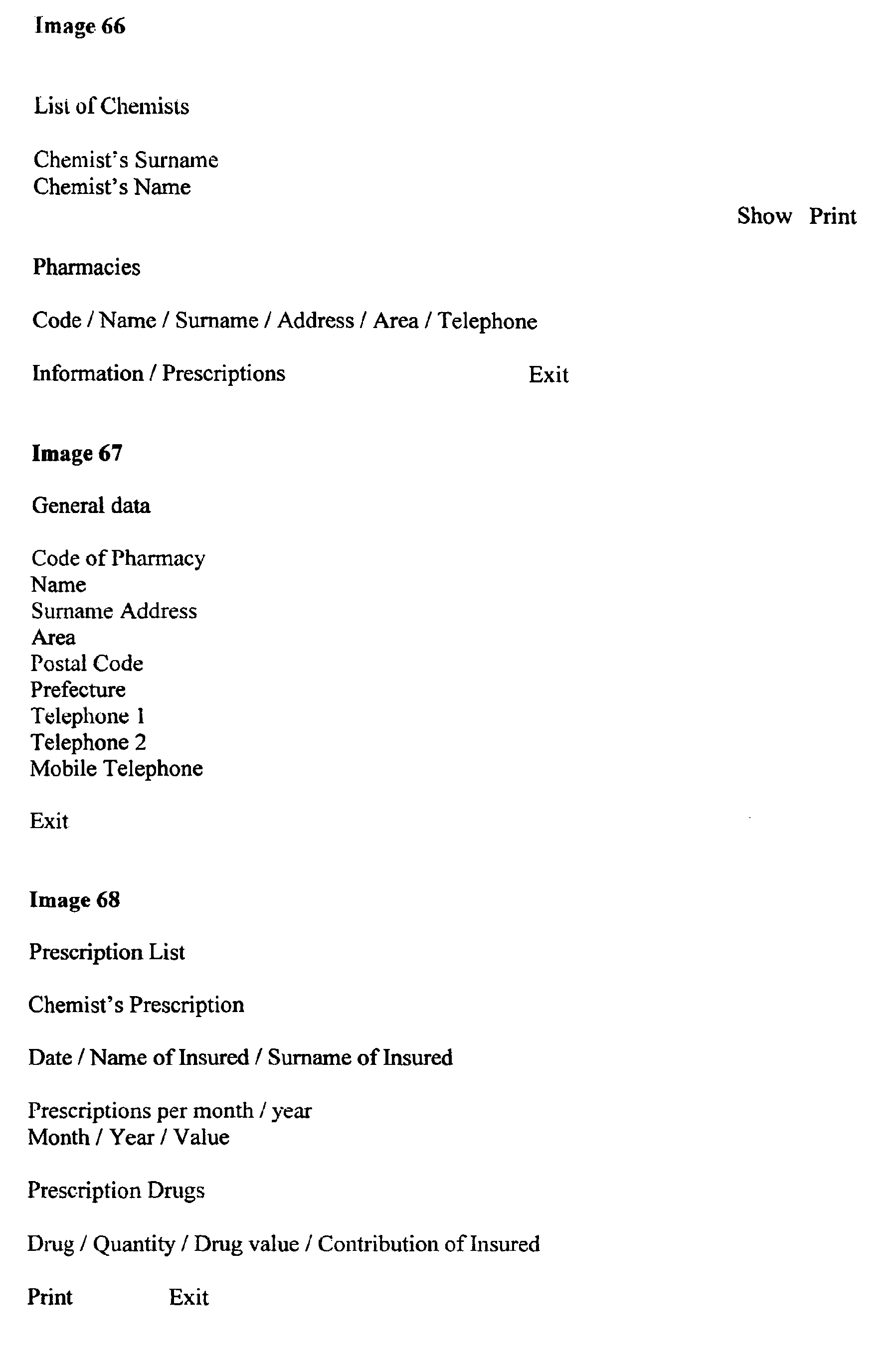

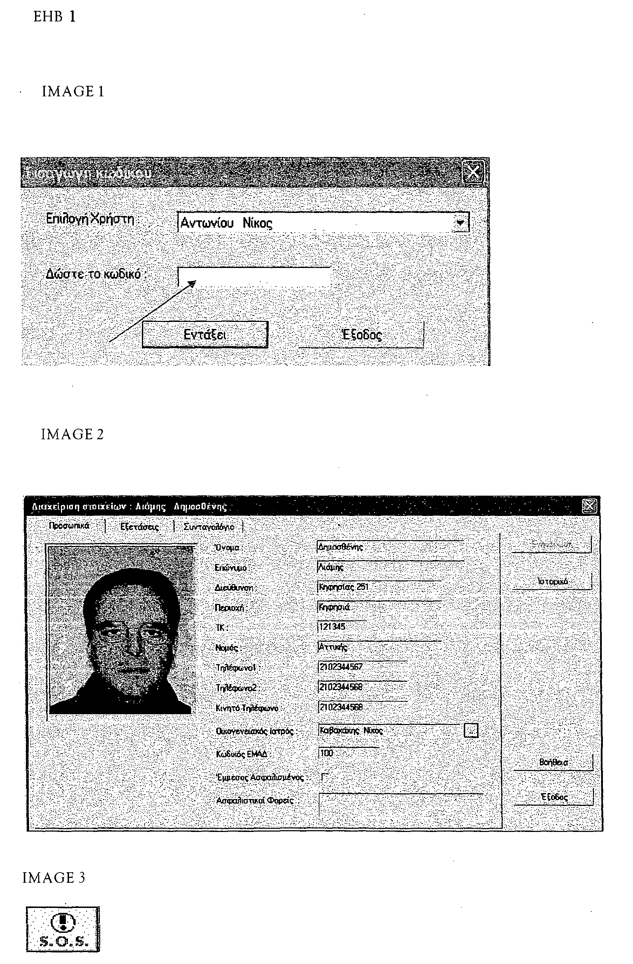

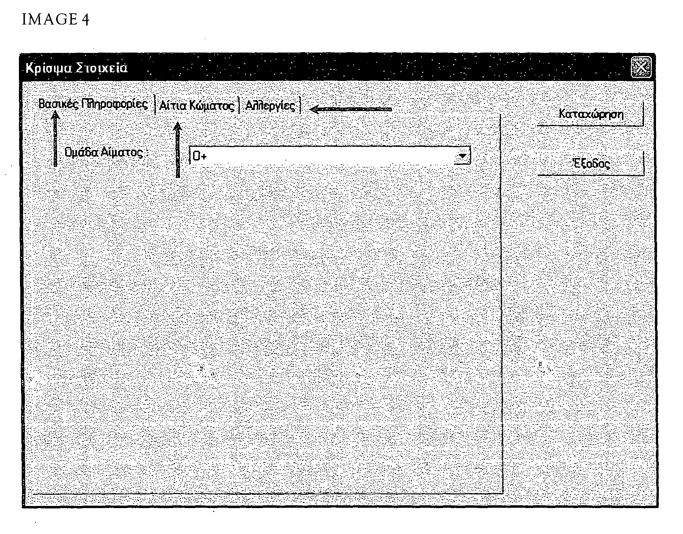

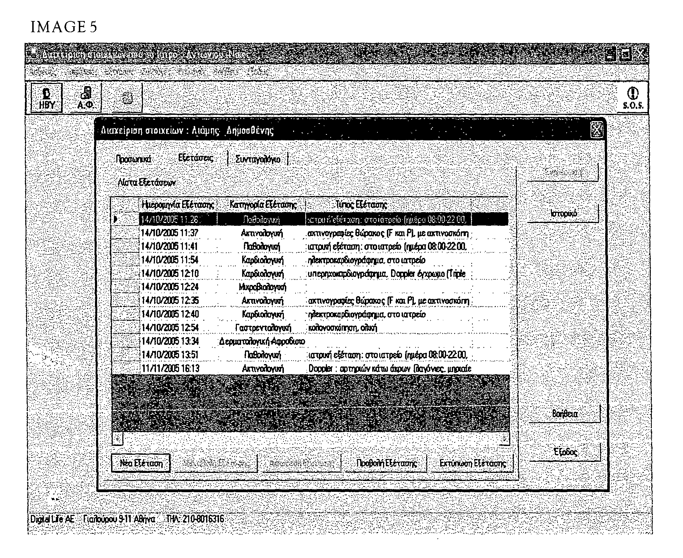

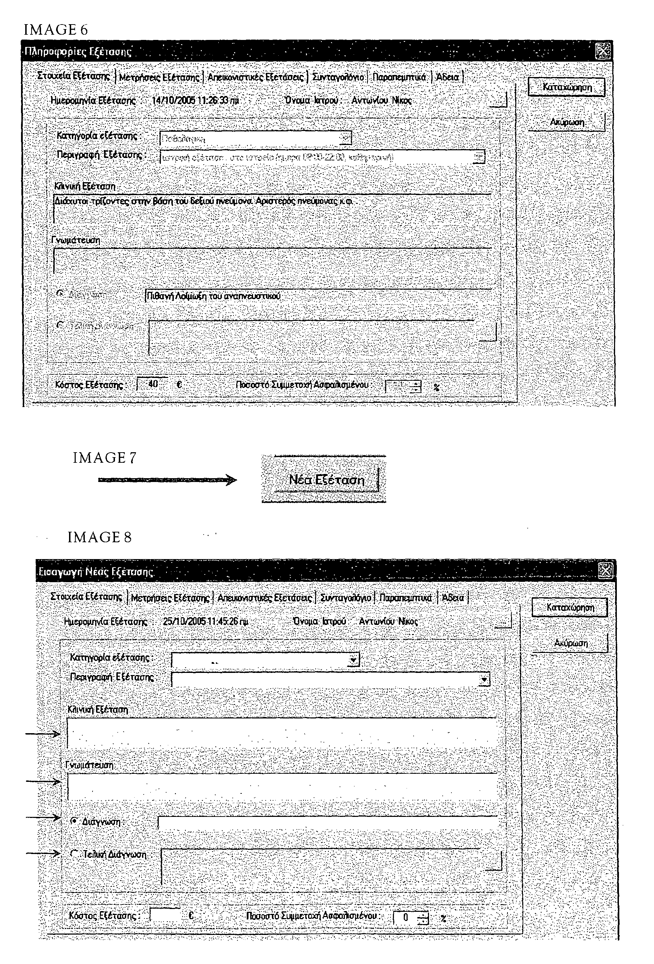

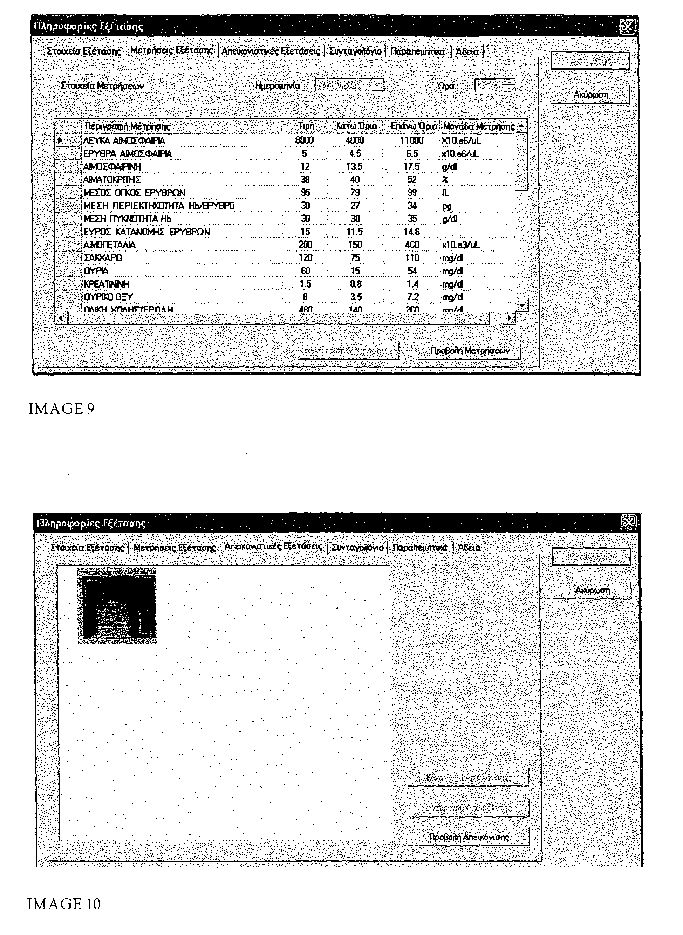

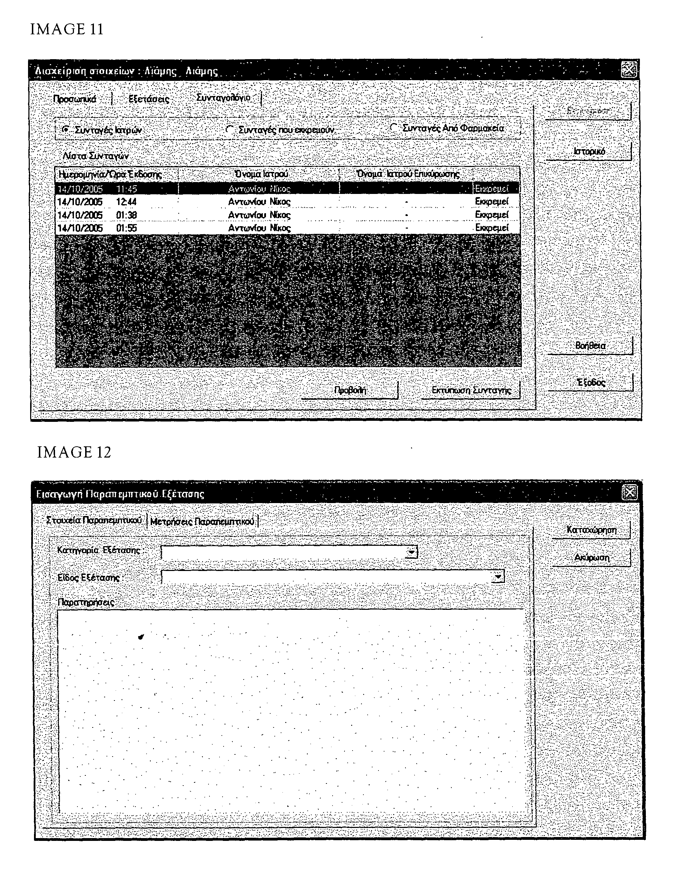

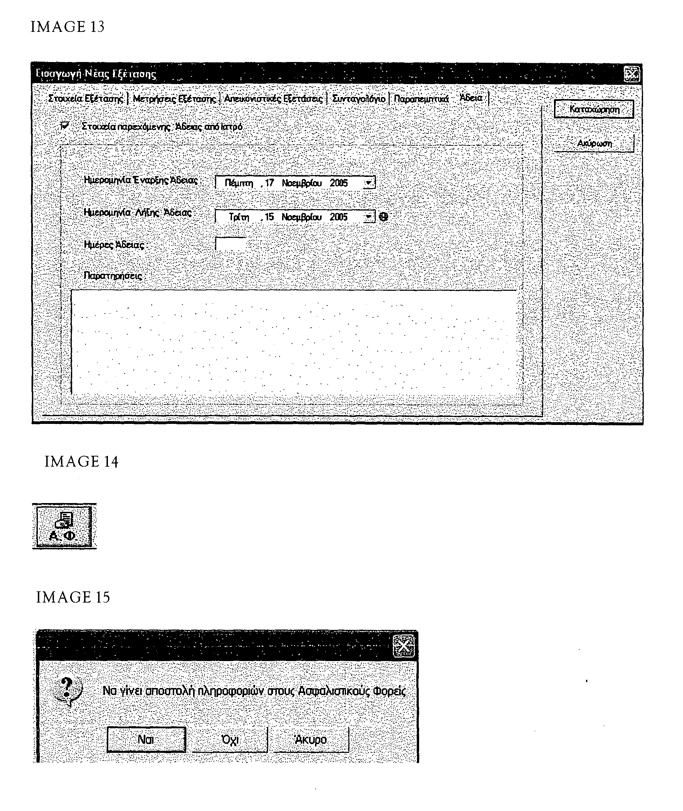

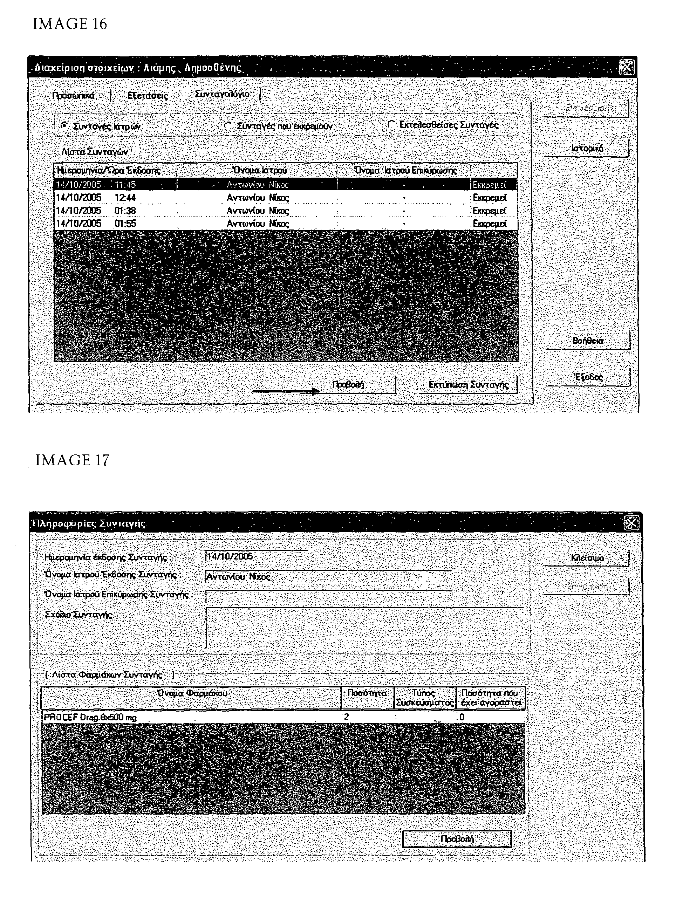

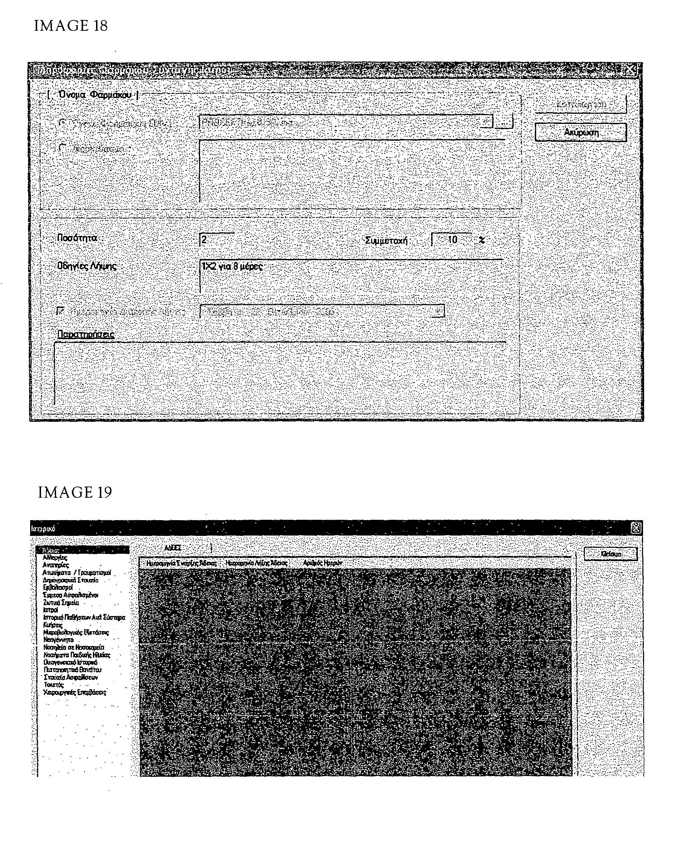

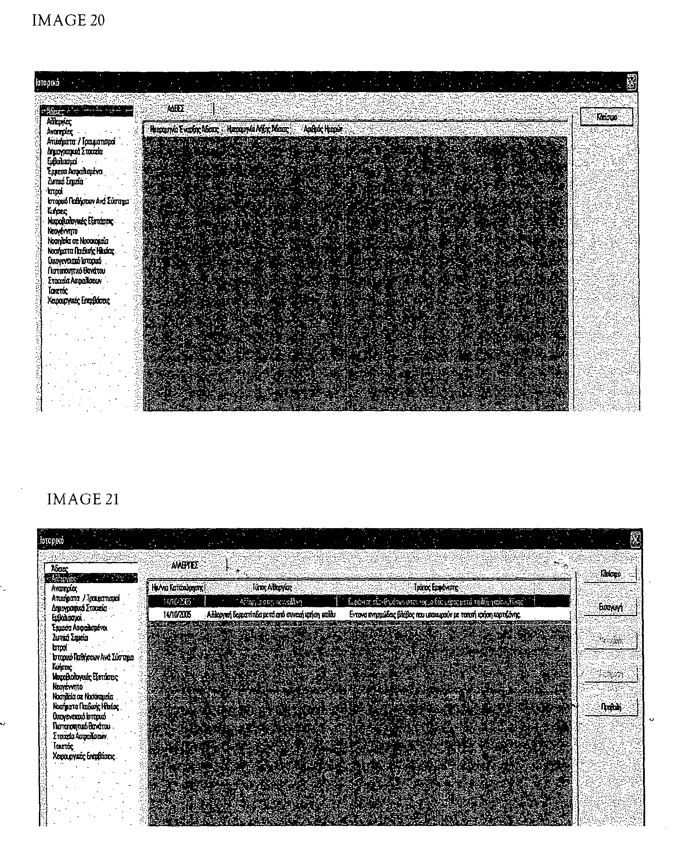

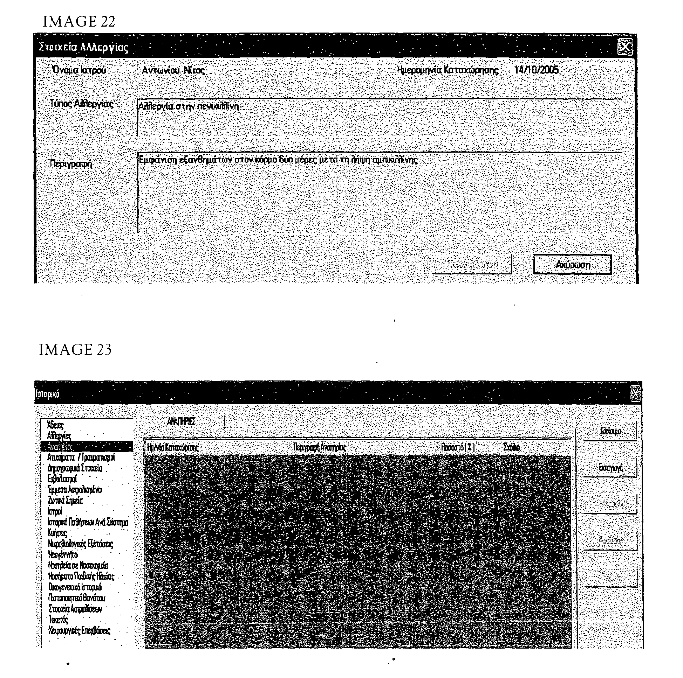

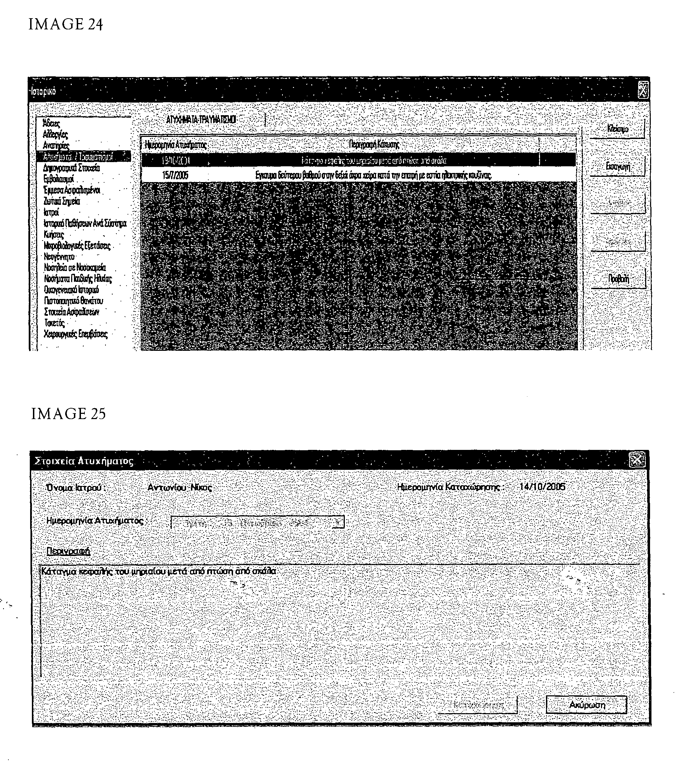

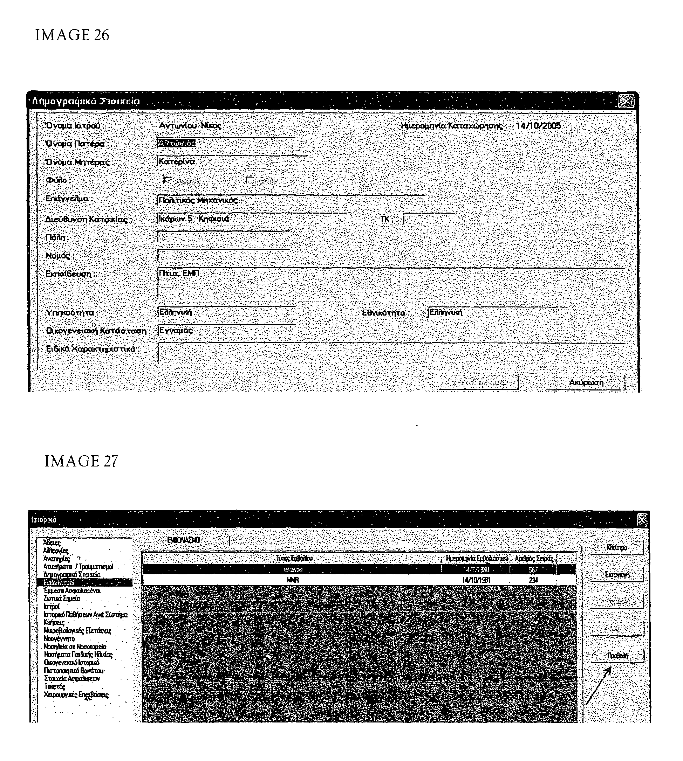

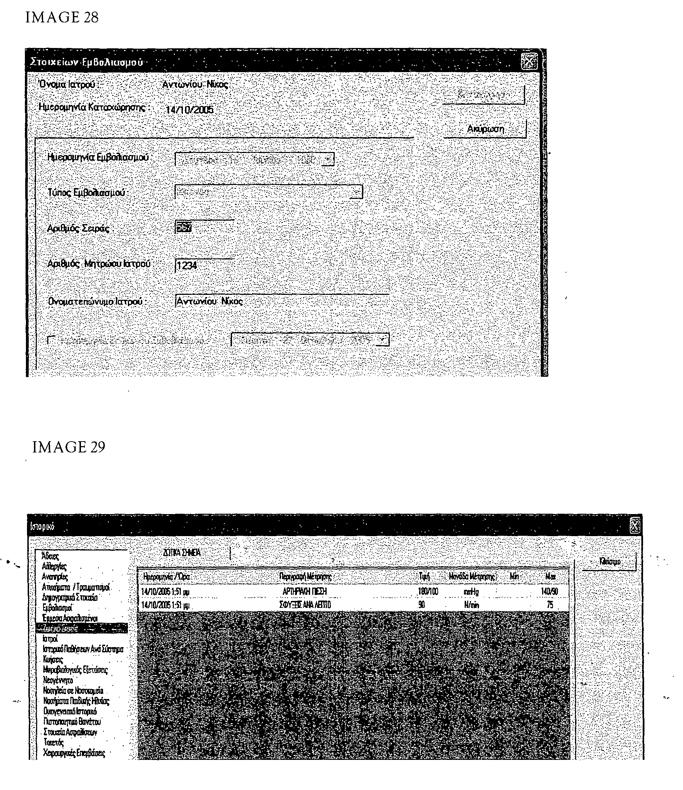

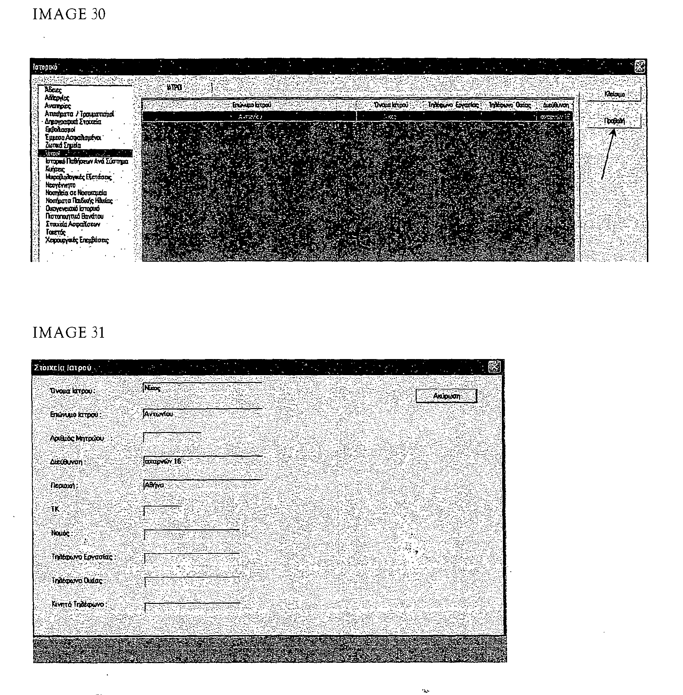

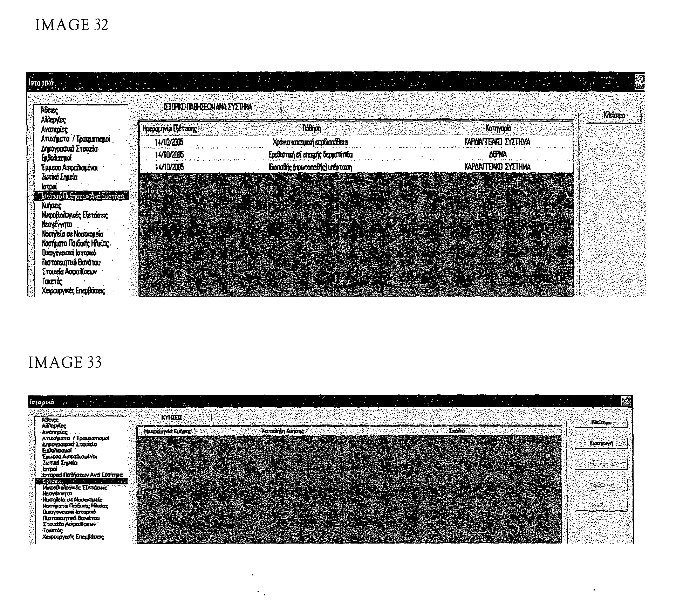

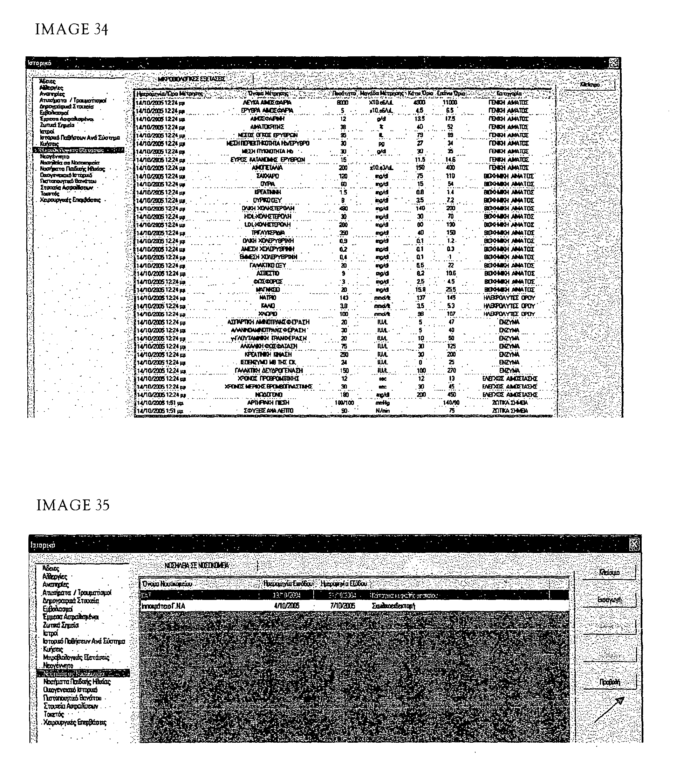

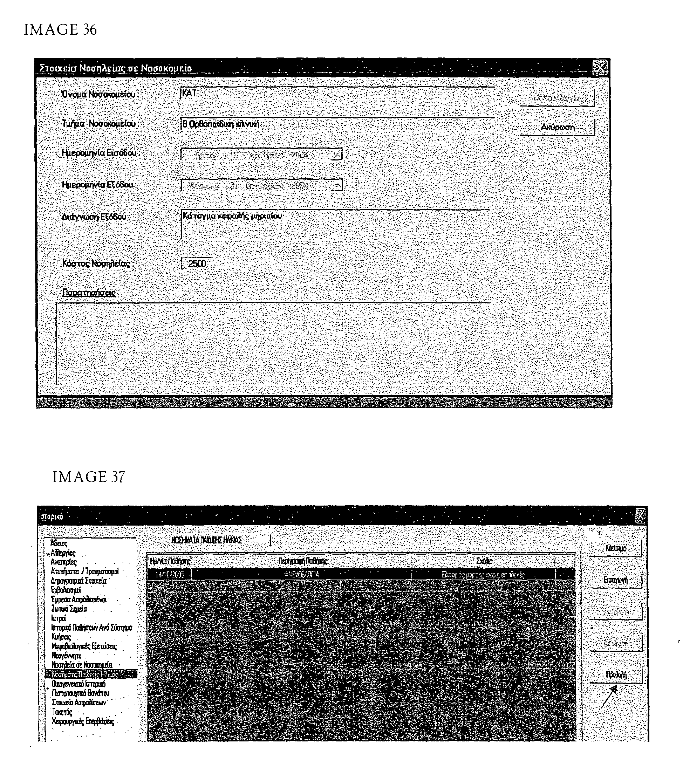

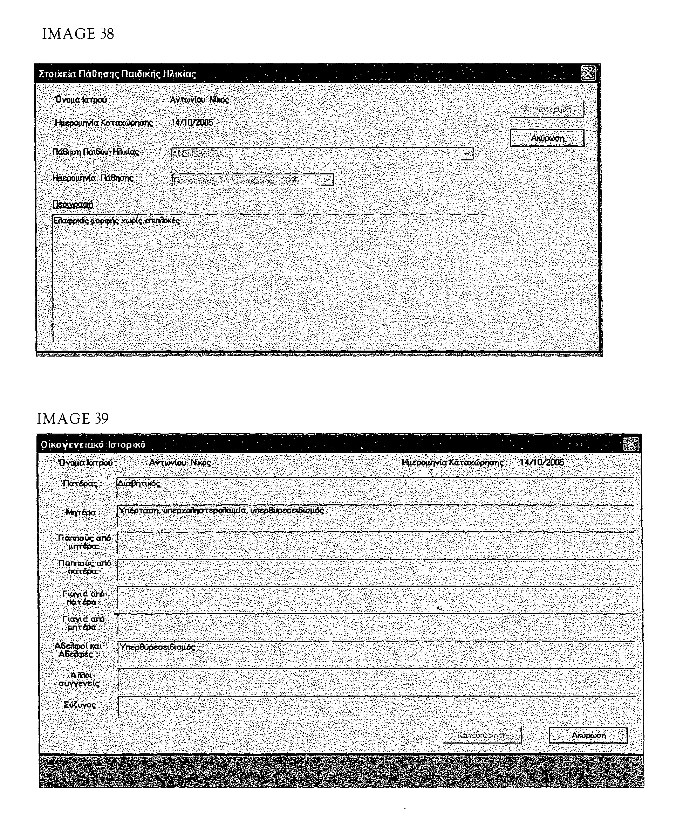

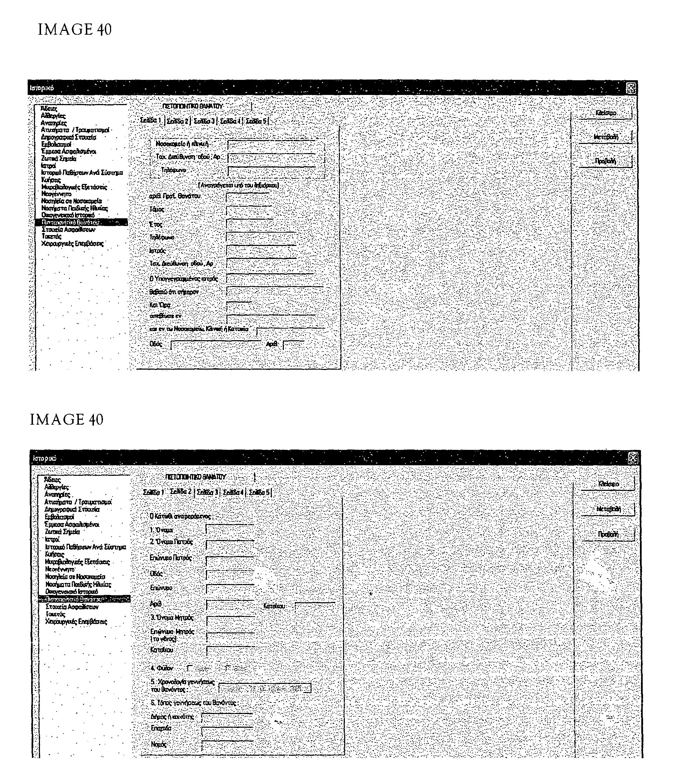

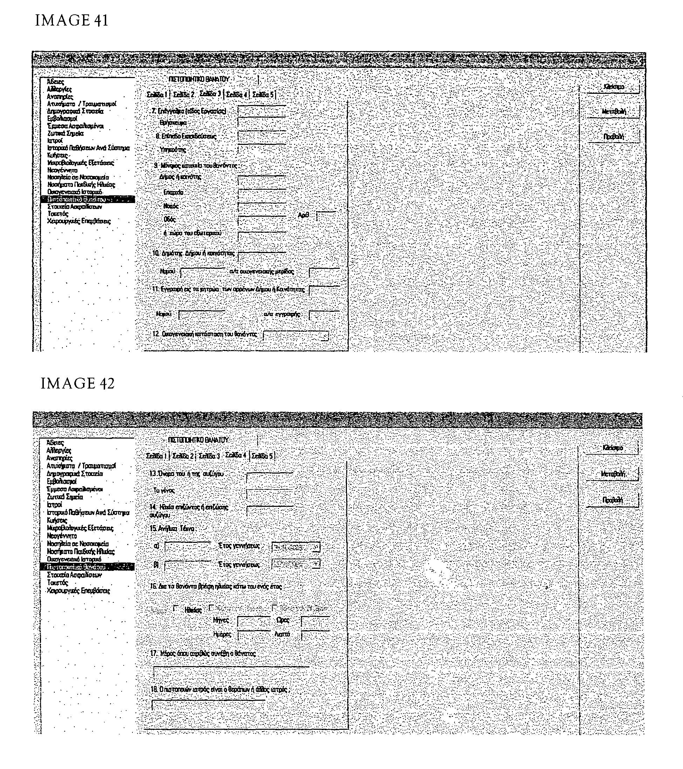

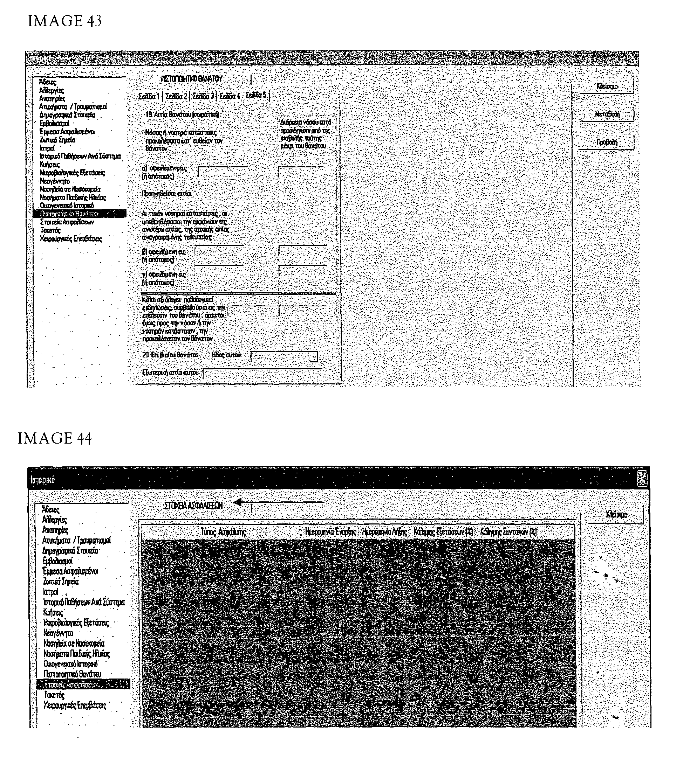

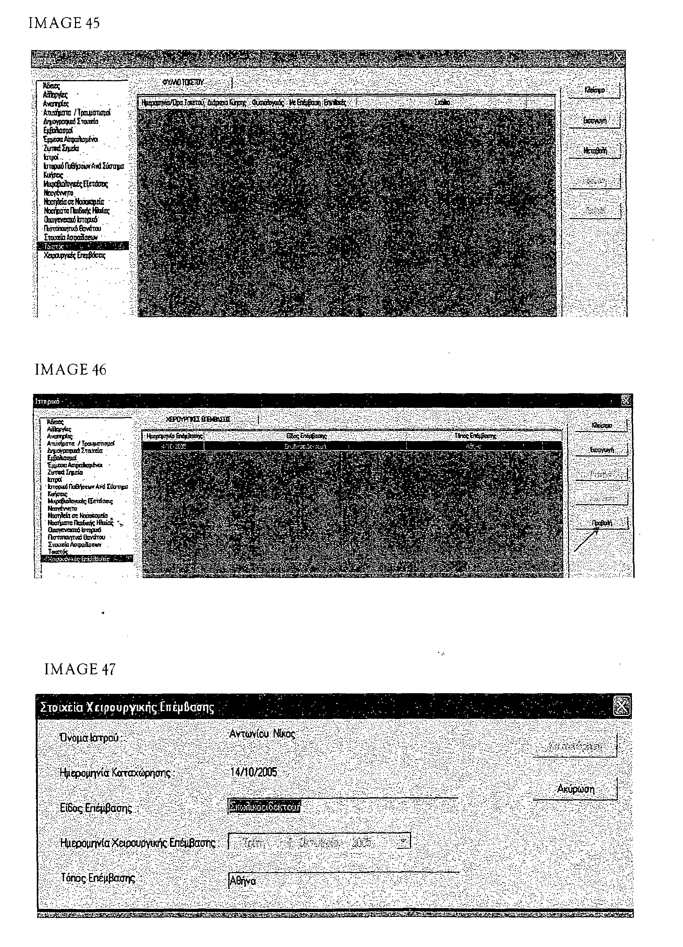

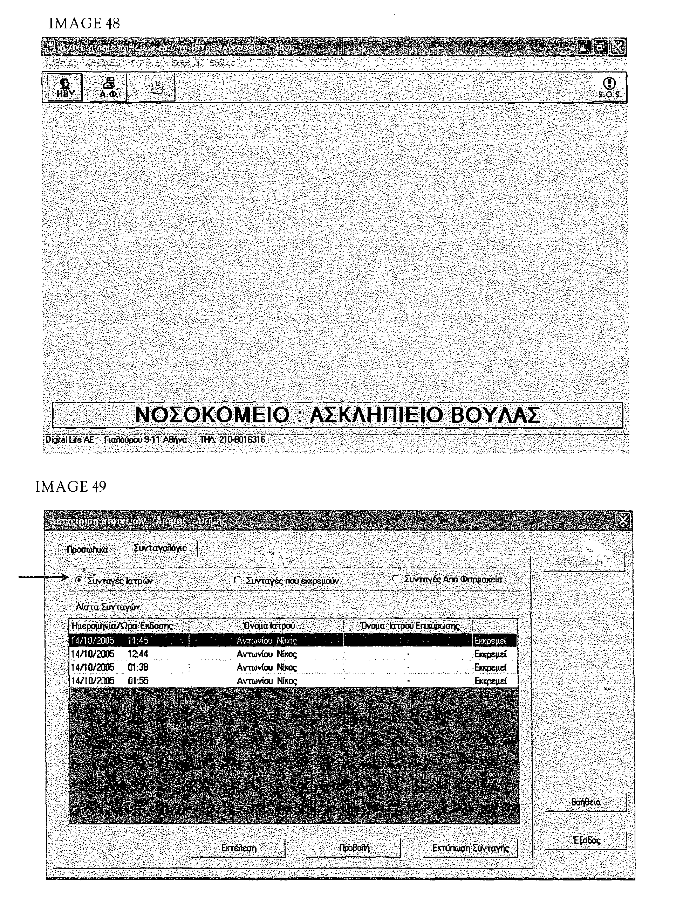

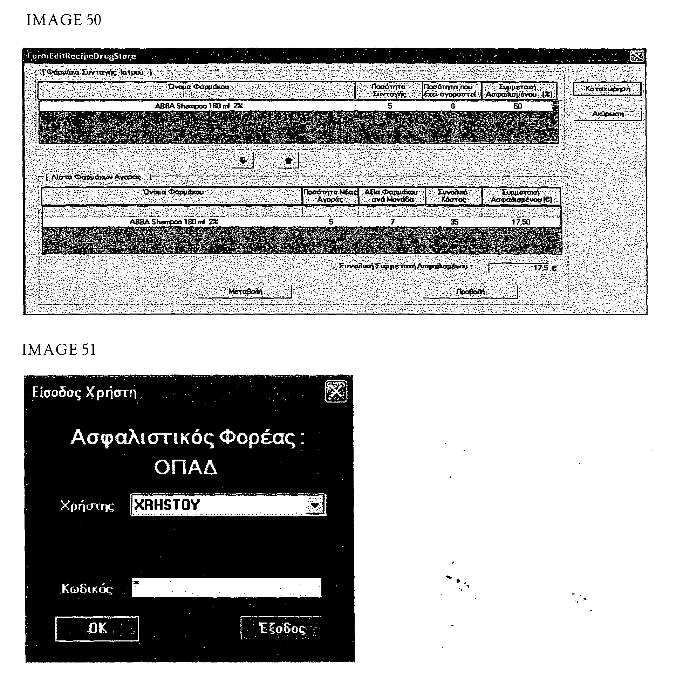

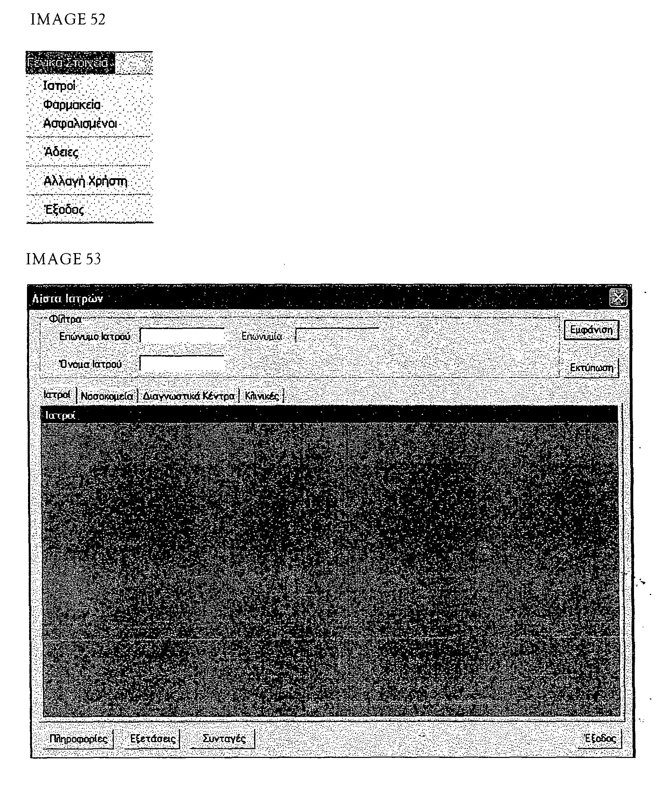

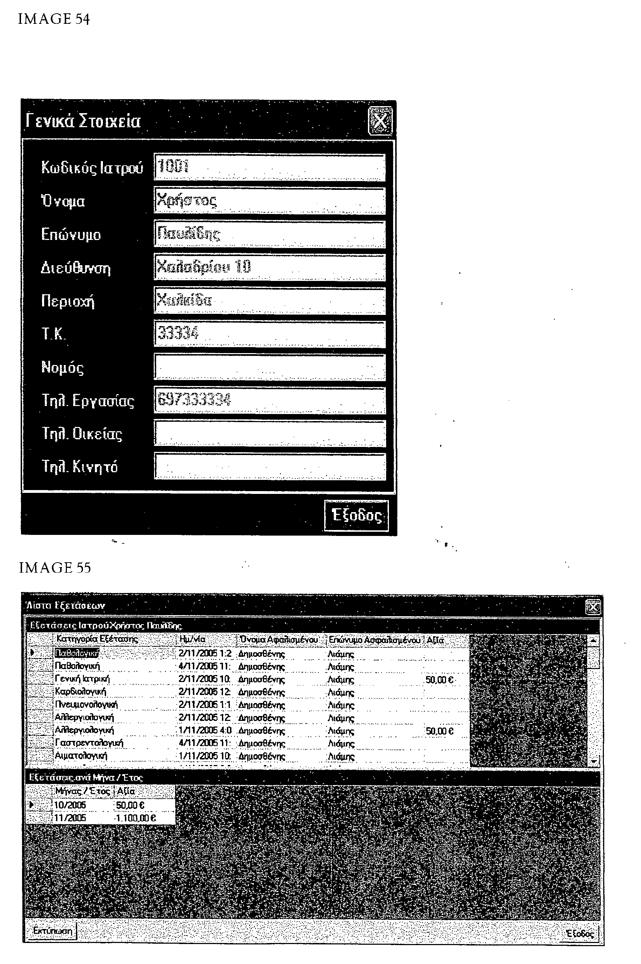

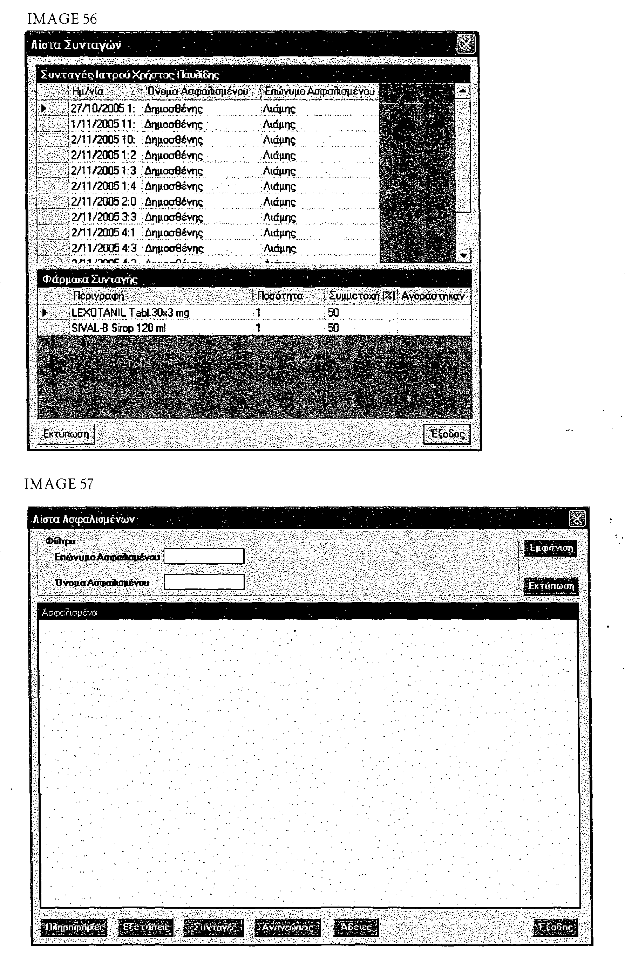

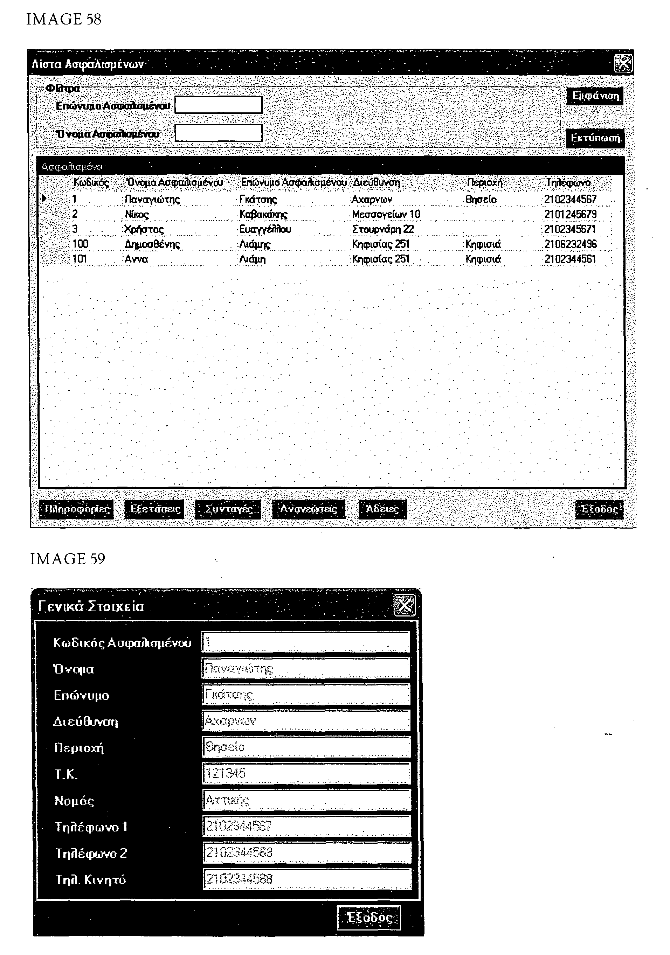

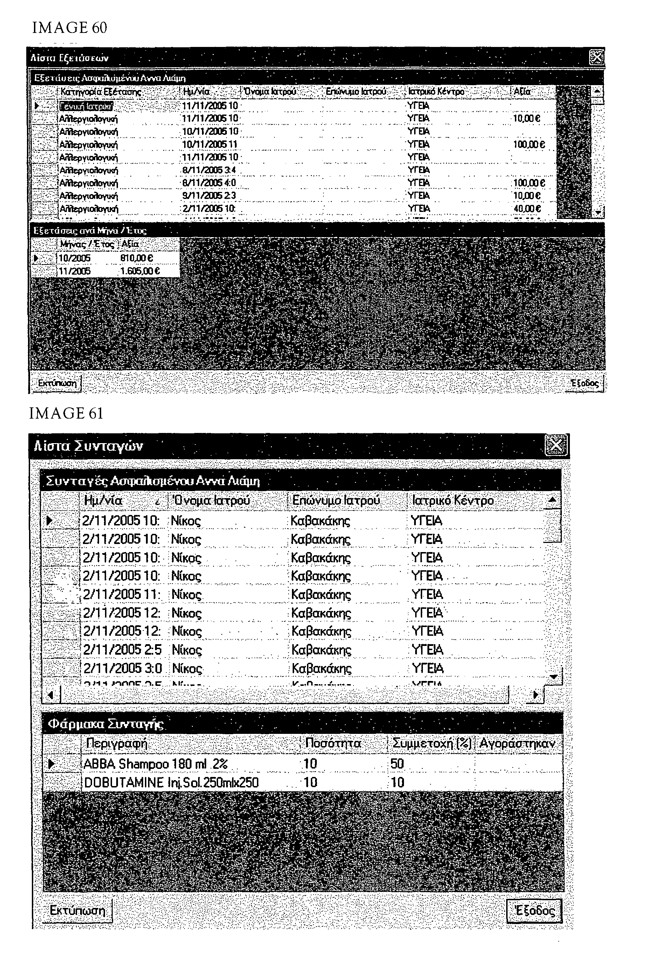

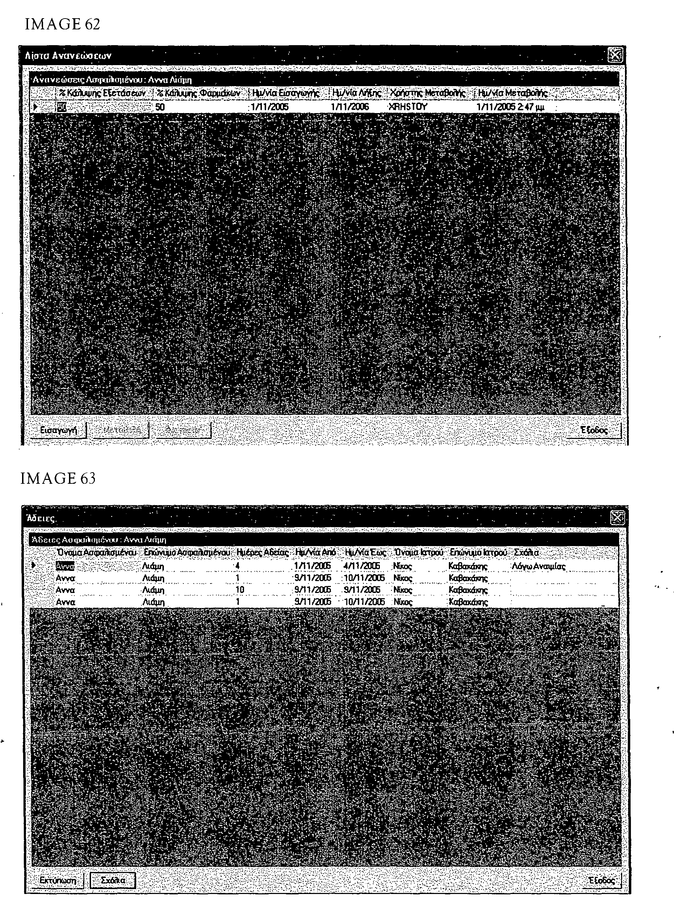

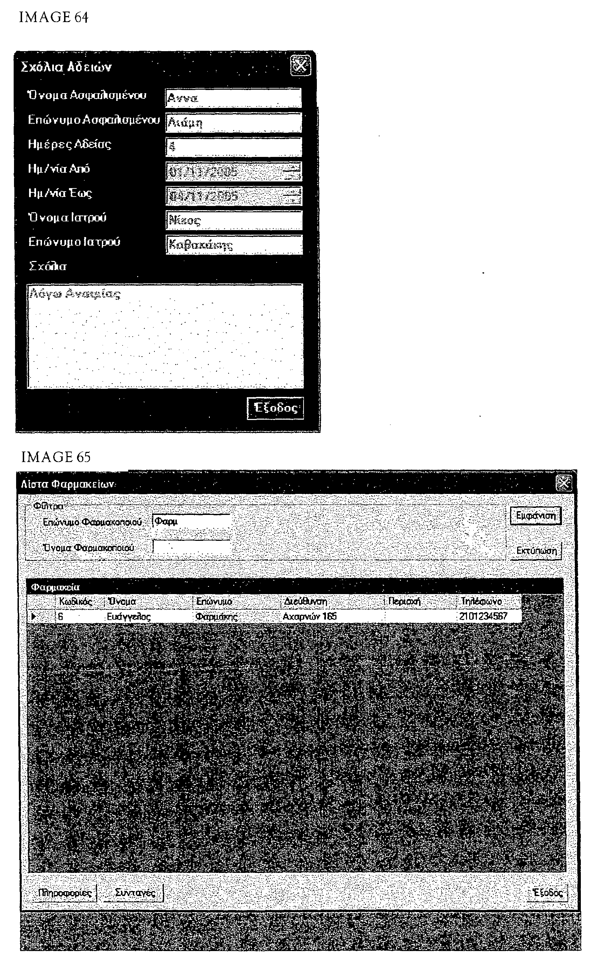

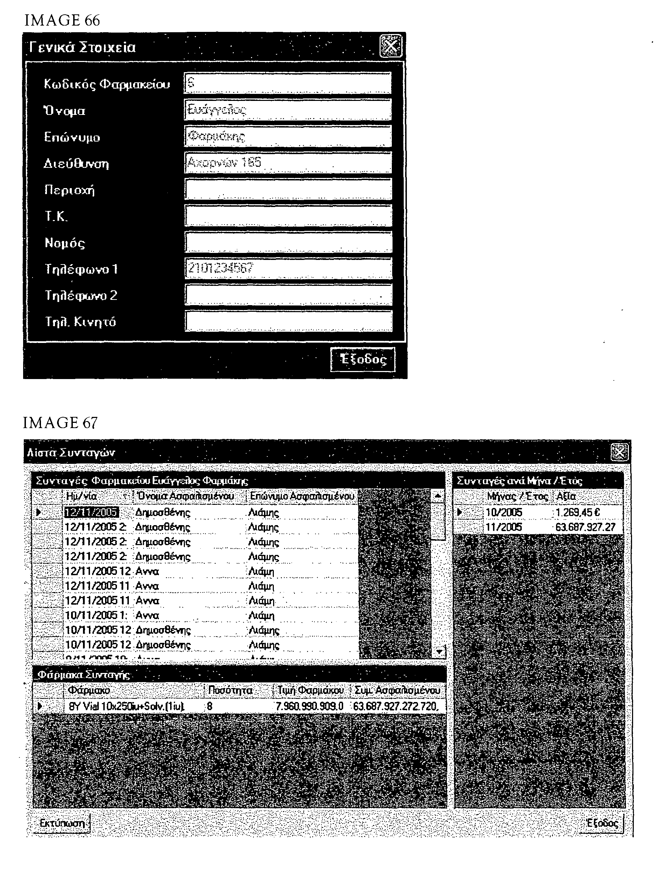

## Claims

1. The Electronic Health Book consists of a full operating system that covers the entire system of health, Public and private, having as a center the citizen (holder) and as knots: the Doctor, the Hospital, the Diagnostic center, the drugstore and finally the insurance institution. It uses as storage means an electronic means of large capacity usb or cd or any other large capacity means. It is **characterized** from the fact that it has the capability to store a large volume of medical information regarding its holder in a form of electronic text, (through special programs), but also medical depictions (radiographs - ultrasound - magnetic etc) all these in fact constitute the full medical history record of the holder, updated by all doctors or hospitals or diagnostic centers, which he visits from time to time. It does not offend personal data because the citizen has it in his full possession, it is encoded, and the doctor's identity when during the visit he submits it to him, it is certified through the system via processes which ensure absolute security for personal data. There is no central medical database from which sensitive medical data of any citizen could transpire. Capability is provided for communication to be conducted by a doctor, hospital, diagnostic center and drugstore with the insurance institution, which covers the holder, through the internet by the use of special programs, so the charges of the medical actions are transferred electronically and the doctors, hospitals, drugstores are compensated for immediately following a full and immediate automated audit. The doctor can after he receive the EHB from the holder to read the entire medical history record of the holder (it is covered by medical secrecy) and to add new examinations, opinions, and prescriptions, leave, as well as financial data regarding medical actions, in electronic form. The pharmacist **cannot read medical information with the exception only of the doctor's prescription** and to execute it electronically, simply by pressing the specific program option.

2. Electronic equipment configuration for reading and recording the electronic card mentioned in the above claim 1, **characterized by** the fact that it allows, with the assistance of special software, the following services:
A. Electronic configuration of the card so that the storage of data listed in the above claim 1 is possible, in encrypted form
B. Reading the card holder's data by medical personnel for conducting hospitalization and appropriate charging of the holder depending on his insurance coverage
C. Renewal of medical history record of the patient by medical personnel with an appropriate card entry
D. Renewal of the cardholder's insurance data by the insurance institution (capability of conducting this service also through the internet).
E. Production of an encrypted file that contains the necessary data of the cardholder and of the hospitalization data for the compensation of the doctor / hospital (capability of dispatching the file and carrying out the payment electronically as well, through the internet).

3. The Electronic Health Book according to claims 1 and 2 is **characterized by** the fact that it obtains the capability of transferring all selected medical data of the holder by the **Doctor user to Medical centers** supporting it through Remote Medicine, through special operation, so that the difficult Medical incidents are handled with success.

4. The Electronic Health Book according to claims 1, 2, and 3 is **characterized by** the fact that it has the capability of tracking it down, through a transmitter-receiver system, so that it is tracked down with ease. This is achieved with the addition of an audio-signal receiver, which is placed in the case of the EHB.
